(19) Europäisches Patentamt
European Patent Office
Office européen des brevets

(11) Publication number: **0 127 182**
**B1**

(12) **EUROPEAN PATENT SPECIFICATION**

(45) Date of publication of patent specification: **30.12.86**

(21) Application number: **84106160.9**

(22) Date of filing: **30.05.84**

(51) Int. Cl.⁴: **C 07 D 295/08,**
C 07 D 241/04,
C 07 D 243/08,
A 61 K 31/495, A 61 K 31/55

(54) **1-Phenylalkyl-4-(mercapto and carbamylthio)alkyl-piperazines and -homopiperazines and their use in the treatment of allergic diseases.**

(30) Priority: **31.05.83 US 499188**

(43) Date of publication of application:
**05.12.84 Bulletin 84/49**

(45) Publication of the grant of the patent:
**30.12.86 Bulletin 86/52**

(84) Designated Contracting States:
**AT BE CH DE FR GB IT LI LU NL SE**

(56) References cited:
**FR-A-1 286 198**
**GB-A- 952 142**
**JP-A-68 013 468**

(73) Proprietor: **Boehringer Ingelheim Ltd.**
**90 East Ridge P.O. Box 368**
**Ridgefield, Conn 06877 (US)**

(72) Inventor: **Hargrave, Karl D., Dr**
**16 Willow Run**
**BrookfieldCenter Connecticut 06805 (US)**
Inventor: **Devlin, John P., Dr.**
**57 Montgomery Street**
**Poughkeepsie New York 12601 (US)**
Inventor: **Barsumian, Edward L., Dr.**
**55 Mill Plain Road**
**Danbury Connecticut 06810 (US)**

(74) Representative: **Laudien, Dieter, Dr.**
**Boehringer Ingelheim Zentrale GmbH ZA**
**Patente Postfach 200**
**D-6507 Ingelheim am Rhein (DE)**

**EP 0 127 182 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel substituted phenylalkyl(piperazinyl or homopiperazinyl)alkyl-thiols and -thiocarbamates and non-toxic acid addition salts thereof, to a method of preparing these compounds, to pharmaceutical compositions containing them as active ingredients, and to a method of using them as therapeutics. By virtue of their potent inhibition of mediator release in numerous cell systems, the compounds are useful for the treatment of immunological, inflammatory and allergic disorders.

The Prior Art

Japanese Patent No. 68 13 468, abstracted in C.A. 70, 11720f (1969), discloses 2-[4-(2-methylbenzyl)-piperazin-1-yl]ethanethiol dihydrochloride as a synthetic intermediate in the preparation of sedative and antiulcer drugs.

The compound 2-(4-benzylpiperazin-1-yl)-1-methylethanethiol has a Chemical Abstract Registry Number (82627-25-6), but has not yet appeared in the literature.

German Offenlegungsschrift 2,551,355 discloses 2-[4-(2-aminobenzyl)piperazin-1-yl]-2-oxo-ethanethiol and 2-[4-(2-amino-3,5-dibromobenzyl)piperazin-1-yl)-2-oxo-ethanethiol as antihistaminics and anticonvulsants.

The Invention

More particularly, the present invention relates to novel substituted phenylalkyl(piperazinyl or homopiperazinyl)alkylthiols or-thiocarbamates represented by the formula

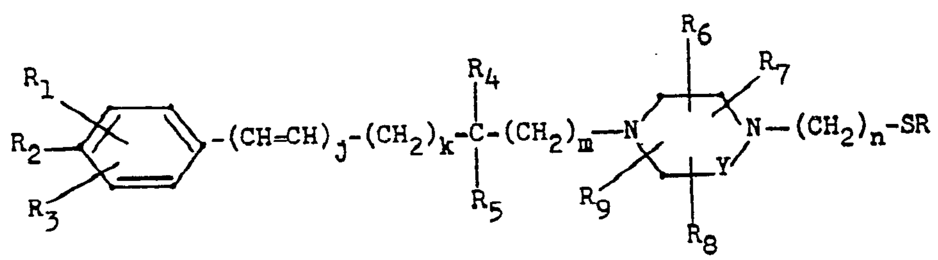

(I)

wherein

R is hydrogen or

O
||
—C—NH—A;

A is alkyl of 1 to 8 carbon atoms; cycloalkyl of 3 to 7 carbon atoms; or unsubstituted or mono-, di- or trisubstituted phenyl, where the substituents are each alkyl of 1 to 4 carbon atoms, halogen, trihalomethyl, alkoxy of 1 to 3 carbon atoms, carboxylic acyl of 1 to 3 carbon atoms, carboxyl, (alkoxy of 1 to 3 carbon atoms) carbonyl, nitro, cyano or di(alkyl of 1 to 3 carbon atoms)amino;

$R_1$, $R_2$ and $R_3$, which may be identical to or different from each other, are each hydrogen, halogen, alkyl of 1 to 4 carbon atoms, triahlomethyl, nitro, cyano, di(alkyl of 1 to 4 carbon atoms)amino, (alkoxy of 1 to 4 carbon atoms) carbonyl, alkoxy of 1 to 4 carbon atoms or hydroxyl;

$R_4$ and $R_5$, which may be indentical to or different from each other, are each hydrogen, alkyl of 1 to 4 carbon atoms or phenyl;

$R_6$, $R_7$, $R_8$ and $R_9$, which may be identical to or different from each other, are each hydrogen or methyl;

Y is $—CH_2—$ or $—CH_2—CH_2—$;

j is 0 or 1;

K and m are each integers of 0 to 3, their sum being no more than 6 and must be 0 when j is 1;

n is an integer of 2 to 4, with the proviso that n must be 3 or 4 when $R_1$ is hydrogen or 2-methyl, $R_2$, $R_3$, $R_4$, $R_5$, $R_6$, $R_7$, $R_8$, $R_9$ and R are hydrogen and j, k and m are O, and Y is $—CH_2—$;

and non-toxic, pharmaceutically acceptable acid addition salts thereof.

Specific examples of variables A, $R_1$, $R_2$, $R_3$, $R_4$ and $R_5$ are the following:

A — Methyl, ethyl, propyl, butyl, pentyl, hexyl, heptyl, octyl or possible isomers thereof; cyclopropyl, cyclobutyl, cyclopentyl, cyclohexyl, or cycloheptyl. The optional substitutents on the phenyl group: Methyl, ethyl, propyl, butyl or possible isomers thereof; fluorine, chlorine or bromine; trifluoromethyl or trichloromethyl; methoxy, ethoxy, propoxy, butoxy or possible isomers thereof; acetyl, propionyl or butyryl; methoxycarbonyl, ethoxycarbonyl or propoxycarbonyl; dimethylamino, N-methylethylamino, diethylamino, N-ethyl-propylamino, N-methyl-propylamino or dipropylamino.

$R_1$, $R_2$ and $R_3$ — Fluorine, chlorine or bromine; methyl, ethyl, propyl, butyl or a possible isomer thereof; trifluoromethyl or trichloromethyl; dimethylamino, N-methyl-ethylamino, diethylamino, N-ethyl-propylamino, N-methyl-propylamino or dipropylamino; methoxycarbonyl, ethoxycarbonyl or propoxycarbonyl; methoxy, ethoxy, propoxy, butoxy or possible isomers thereof.

$R_4$ and $R_5$ — Methyl, ethyl, propyl or isopropyl.

A preferred subgenus thereunder is constituted by compounds of the formula

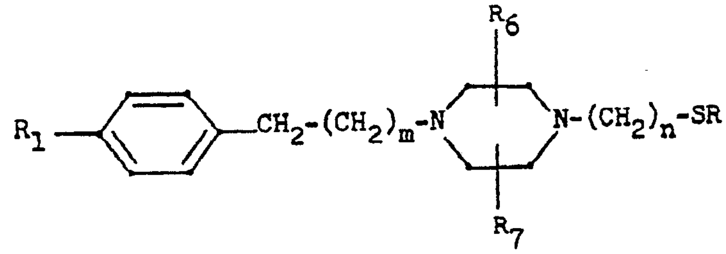

(Ia)

wherein

R is hydrogen or

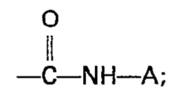

A is alkyl of 1 to 8 carbon atoms; cycloalkyl of 3 to 7 carbon atoms; or unsubstituted or mono-, di- or trisubstituted phenyl, where the substituents are each indepedendently alkyl of 1 to 4 carbon atoms, halogen, trihalomethyl, alkoxy of 1 to 3 carbon atoms, carboxylic acyl of 1 to 3 carbon atoms, carboxyl, (alkoxy of 1 to 3 carbon atoms) carbonyl, nitro, cyano or di(alkyl of 1 to 3 carbon atoms)amino;

$R_1$ is hydrogen, halogen, alkyl of 1 to 4 carbon atoms, trihalomethyl, nitro, cyano, di(alkyl of 1 to 4 carbon atoms)amino, (alkoxy of 1 to 4 carbon atoms)carbonyl, alkoxy of 1 to 4 carbon atoms or hydroxyl;

$R_6$ and $R_7$ are each indepedently hydrogen or methyl;

m is an integer from 0 to 3; and

n is an integer of 2 to 4, with the proviso that n must be 3 or 4, when $R_1$ is hydrogen or 2-methyl, $R_6$, $R_7$ and R are hydrogen and m is O;

and non-toxic, pharmaceutically acceptable acid addition salts thereof.

An especially preferred subgenus is constituted by compounds of the formula Ia, where

R is hydrogen or

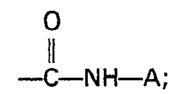

A is alkyl of 1 to 3 carbon atoms; cycloalkyl of 5 to 6 carbon atoms; or unsubstituted or mono-, di- or trisubstituted phenyl, where the substituents are each independently alkyl of 1 to 4 carbon atoms, fluorine, chlorine, bromine, trifluoromethyl, methoxy, acetyl or carbethoxy;

$R_1$ is hydrogen, fluorine, chlorine, bromine, alkyl of 1 to 4 carbon atoms, trifluoromethyl or dimethyl-amino;

$R_6$ and $R_7$ are each independently hydrogen or methyl;

m is an integer from 0 to 3; and

n is an integer of 2 to 4, with the proviso that n must be 3 or 4 when $R_1$ is hydrogen or 2-methyl, $R_6$, $R_7$ and R are hydrogen and m is 0;

and non-toxic, pharmaceutically acceptable acid addition salts thereof.

The compounds embraced by formula I may be prepared by reacting a phenylalkyl-piperazine or -homopiperazine of the formula

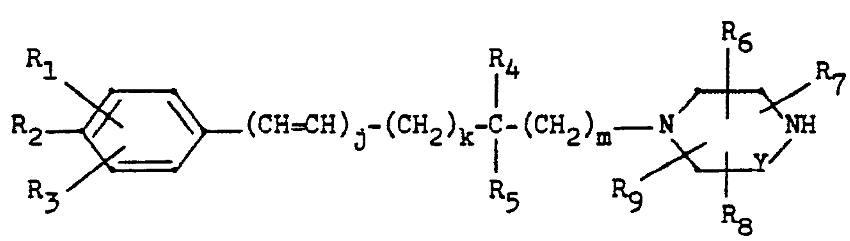

(II)

wherein $R_1$, $R_2$, $R_3$, $R_4$, $R_5$, $R_6$, $R_7$, $R_8$, $R_9$, j, k, m and Y have the meanings previously defined, with an alkyl halide of the formula

$$X—(CH_2)_n—Z \qquad (III)$$

wherein X and Z are identical or different halogens, and n has the meaning previously defined, to provide the alkyl halide of the formula

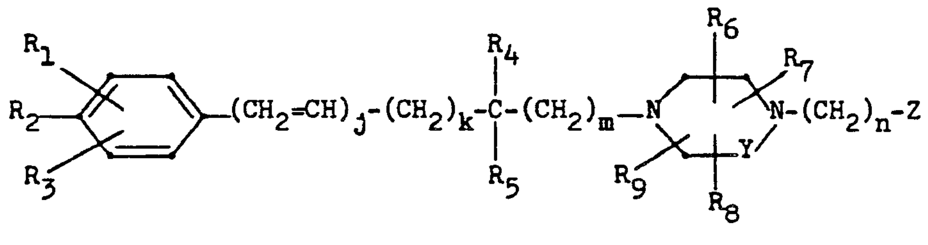

(IV)

wherein $R_1$, $R_2$, $R_3$, $R_4$, $R_5$, $R_6$, $R_7$, $R_8$, $R_9$, Y, Z, j, k, m and n have the measnings previously defined; the alkyl halide IV on reaction with thiourea, followed by hydrolysis of the intermediate isothiouronium halide can provide an end product of the formula wherein R is hydrogen, and, if desired, reacting said end product with an isocyanate of the formula

$$A—N=C=O \quad (V)$$

wherein A has the meanings previously defined, to obtain an end product of the formula I wherein R is —CO—NH—A.

The alkylation reaction described may be performed in a suitable solvent, such as water, dimethyl sulfoxide, dimethyl formamide, a lower alkanol of up to five carbon atoms, tetrahydrofuran or acetone, in the presence of a strong inorganic or organic base such as sodium or potassium hydroxide, a trialkylamine or pyridine and at room or elevated temperature up to the boling point of the reaction mixture. The reaction time is temperature-dependent and may be one to several hours.

The subsequent reaction of phenylalkyl piperazinylalkyl halide of the formula IV with thiourea is carried out in the same solvents and bases as those mentioned in connection with the alkylation process; the reaction temperature may be up to the boiling point of the reaction mixture. Hydrolysis of the intermediate isothiouronium halide may be effected by the addition of water and strong inorganic base, e.g. sodium or potassium hydroxide at room or elevated temperature up to the boiling point of the reaction mixture. With this reaction free thiols of the formula I are obtained.

For the preparation of an end product wherein R is —CO—NH—A, an above-mentioned thiol is reacted with an isocyanate of the formula V in the presence of a suitable inert solvent such a dioxane, tetrahydrofuran, ether, toluene or chlorinated hydrocarbons and optionally in the presence of an inorganic or organic base such as sodium or potassium carbonate, a trialkylamine or pyridine.

The temperature may arise up to a reflux while the reaction time depends on the starting material and temperature used and may last from 30 minutes to several hours.

The compounds embraced by formula I are basic and therefore form addition salts with inorganic or organic acids. Examples of non-toxic, pharmacologically acceptable acid addition salts are those formed with hydrohalic acid, especially hydrochloric or hydrobromic acid, nitric acid, sulfuric acid, o-phosphoric acid, citric acid, maleic acid, fumaric acid, propionic acid, butyric acid, acetic acid, succinic acid, methanesulfonic acid, benzenesulfonic acid, p-toluenesulfonic acid, or the like.

The starting compounds are known or may be prepared by known methods.

For example, compounds of the formula II are described in British Patent No. 480,358 and J. Am. Chem. Soc. *66,* 263 (1944).

Compounds of the formula IV are known, for example, from Helv. Chim. Acta *41,* 1072 (1958) and Monatshefte *87,* 701 (1956).

The following examples illustrate the present invention and will enable others skilled in the art to understand it more completely. It should be understod, however, that the invention is not limited solely to the particular examples given below.

Example 1

3-(4-Benzylpiperazin-1-yl)propanethiol dihydrochloride

(a) 3.15 g of 1-bromo-3-chloropropane were added to a mixture of 35 g of 1-benzylpiperazine, 150 ml of dimethyl sulfoxide and 25 g of potassium hydroxide.

The resulting mixture was stirred at room temperature for 3 hours. Water was added to the resulting solution, the reaction product was extracted with ether, dried (magnesium sulfate), and the salt was precipitated with ethereal hydrochloric acid to give 35.6 g (55% of theory) of 1-benzyl-4-(3-chloropropyl)-piperazine dihydrochloride as a white crystalline solid.

(b) 15 g of thiourea were added to a solution of 35 g of 1-benzyl-4-(3-chloropropyl)piperazine dihydrochloride, 20 g of triethylamine and 250 ml of reagent ethanol, and the mixture was refluxed for 8 hours. After the addition of a solution of 10 g of sodium hydroxide in 50 ml of water, the resulting mixture was

refluxed for 4 additional hours. The ethanol was removed in vacuo, and water added to the residue which was extracted with methylene chloride, dired (sodium sulfate), and the salt was precipitated with ethereal hydrochloric acid to give 25.1 g (78% of theory) of 3-(4-benzyl-piperazin-1-yl)propanethiol dihydrochloride as a white crystalline solid, M.p. 270—273°C (dec.).

Example 2

N-Phenyl-S-[3-(4-benzylpiperazin-1-yl)propyl]thiocarbamate dihydrochloride monohydrate

1.6 g of phenyl isocyanate were added to a solution of 4.0 g of 3-(4-benzylpiperazin-1-yl)propanethiol di-hydrochloride and 2.6 g of triethylamine in 50 ml of methylene chloride. The mixture was refluxed for four hours, washed with water and dried (sodium sulfate). Precipitation of the salt with ethereal hydrochloric acid gave 1.2 g (20% of theory) of N-phenyl-S-[3-(4-benzylpiperazin-1-yl)-propyl]thiocarbamate dihydrochloride monohydrate as a white cyrstalline solid, M.p. 216—219°C.

Example 3

3-[4-(4-Chlorobenzyl)piperazine-1-yl]propanethiol dihydrochloride

(a) Utilizing the procedure described in Example 1(a), 20.0 g of 1-(4-chlorobenzyl)piperazine, 100 ml of dimethyl sulfoxide, 15.0 g of potassium hydroxide and 15.0 of 1-bromo-3-chloropropane yielded 25.1 g (74% of theory) of 1-(4-chlorobenzyl)-4-(3-chloropropyl)piperazine dihydrochloride as a white crystalline solid.

(b) Utilizing the procedure described in Example 1(b), 6.0 g of thiourea, was reacted with 25.2 g of 1-(4-cholorobenzyl-4-(3-chloropropyl)piperazine dihydrochloride and 8.7 g of triethylamine in 200 ml of reagent ethanol. The hydrolysis was effected with 5.0 g of sodium hydroxide in 50 ml of water. Work-up as described above gave 16.6 g (75% of theory) of 3-[4-(4-chlorobenzyl)piperazine-1-yl]propanethiol dihydrochloride as an off-white crystalline solid, M.p. 257—260°C.

Example 4

N-Methyl-S-{3-[4-(4-chlorobenzyl)piperazin-1-yl]-propyl}thiocarbamate dihydrochloride

The procedure described in Example 2 was followed, using 0.4 g of methyl isocyanate, 2.0 g of 3-[4-(4-chloro-benzyl)piperazin-1-yl]propanethiol dihydrochloride, 0.7 g of triethylamine and 50 ml of methylene chloride, to give 0.75 g (32% of theory) of N-methyl-S-{3-[4-(4-chlorobenzyl)piperazine-1-yl]propyl}thiocarbamate dihydrochloride as a white crystalline solid, M.p. 240—243°C.

Example 5

N-Isopropyl-S-{3-[4-(4-chlorobenzyl)piperazin-1-yl-propyl}thiocarbamate dihydrochloride

The procedure described in Example 2 was followed, using 0.5 g of isopropyl isocyanate, 2.0 g of 3-[4-(4-chlorobenzyl)piperazin-1-yl]propanethiol dihydrochloride, 0.7 g of triethylamine and 50 ml of methylene chloride, to give 0.7 g (28% of theory) of N-isopropyl-S-{3-[4-(4-chlorobenzyl)piperazine-1-yl]propyl}thiocarbamate dihydrochloride as a white crystalline solid, M.p. 247—250°C.

Example 6

N-Cyclohexyl-S-{3-[4-(4-chlorobenzyl)piperazin-1-yl]propyl}thiocarbamate dihydrochloride

A mixture of 1.0 g of cyclohexyl isocyanate, 2.1 g of 3-[4-(4-chlorobenzyl)piperazin-1-yl]propanethiol and 10 ml of methylene chloride was stirred at room temperature overnight and then concentrated in vacuo. The residue crystallized upon standing. The product was recrystallized from aqueous ethanol, dissolved in ether, and the ethanol solution was dried (magnesium sulphate). The salt was then precipitated with ethereal hydrochloric acid and recrystallized twice from aqueous ethanol to give 1.2 g (30% of theory) of N-cyclohexyl-S-{3-[4-(4-chlorobenzyl)piperazin-1-yl]propyl}thiocarbamate dihydrochloride as a white crystalline solid, M.p. >165°C (dec.).

Example 7

N-Phenyl-S-{3-[4-(4-chlorobenzyl)piperazin-1-yl]propyl}thiocarbamate dihydrochloride

The procedure described in Example 2 was followed, using 4.3 g of phenyl isocyanate, 12.7 g of 3-[4-(4-chlorobenzyl)piperazin-1-yl]propanethiol dihydrochloride, 7.3 g of triethylamine and 100 ml of methylene chloride, to give 9.1 g (53% theory) of N-phenyl-S-{3-[4-(4-chlorobenzyl)piperazin-1-yl]propyl}thiocarbamate dihydrochloride as a white crystalline solid, M.p. >230°C (dec.).

Example 8

N-(4-n-Butylphenyl)-S-{3-[4-(4-chlorobenzyl)piperazin-1-yl]propyl}thiocarbamate dihydrochloride

The procedure described in Example 2 was followed, using 1.0 g of 4-n-butylphenyl isocyanate, 2.0 g of 3-[4-(4-chlorobenzyl)piperazin-1-yl]propanethiol dihydrochloride, 0.7 g of triethylamine and 50 ml of methylene chloride, to give 0.85 g (28% of theory) of N-(4-n-butylphenyl)-S-{3-[4-(4-chlorobenzyl)-piperazin-1-yl]propyl}thiocarbamate dihydrochloride as a white crystalline solid, M.p. 243—246°C.

Example 9

N-(4-Fluorophenyl)-S-{3-[4-(4-chlorobenzyl)piperazin-1-yl]propyl}thiocarbamate dihydrochloride

The procedure described in Example 2 was followed, using 0.8 g of 4-fluorophenyl isocyanate, 2.2 g of 3-[4-(4-chlorobenzyl)piperazin-1-yl]propanethiol dihydrochloride, 0.6 g of triethylamine and 25 ml of methylene chloride, to give 1.41 g (47% of theory) of N-(4-fluorophenyl)-S-{3-[4-(4-chlorobenzyl)piperazin-1-yl]propyl}thiocarbamate dihydrochloride as a white crystalline solid, M.p. 250—252°C.

Example 10

N-(4-Chlorophenyl)-S-{3-[4-(4-chlorobenzyl)piperazin-1-yl]propyl}thiocarbamate dihydrochloride monohydrate

The procedure described in Example 2 was followed, using 1.5 g of 4-chlorophenyl isocyanate, 3.5 g of 3-[4-(4-chlorobenzyl)piperazin-1-yl]propanethiol dihydrochloride, 1.2 g of trimethylamine and 50 ml of methylene chloride, to give 1.1 g (21% of theory) of N-(4-chlorophenyl)-S-{3-[4-(4-chlorobenzyl)piperazin-1-yl]propyl}thiocarbamate dihydrochloride monohydrate as an off-white crystalline solid, M.p. 243—245°C.

Example 11

N-(3,4-Dichlorophenyl)-S-{3-[4-(4-chlorobenzyl)piperazin-1-yl]propyl}thiocarbamate dihydrochloride

The procedure described in Example 2 was followed, using 1.6 g of 3,4-dichlorophenyl isocyanate, 3.0 g of 3-[4-(4-chlorobenzyl)piperazin-1-yl]propanethio dihydrochloride, 0.9 g of triethylamine and 50 ml of methylene chloride, to give 1.2 g (26% of theory) of N-(3,4-dichlorophenyl)-S-{3-[4-(4-chlorobenzyl)-piperazin-1-yl]propyl}thiocarbamate dihydrochloride as white crystalline solid, M.p. 227—230°C.

Example 12

N-(2-Methoxyphenyl)-S-{3-[4-(4-chlorobenzyl)piperazin-1-yl]propyl}thiocarbamate dihydrochloride sesquihydrate

The procedure described in Example 2 was followed, using 1.3 g of 2-methoxyphenyl isocyanate, 3.0 g of 3-[4-(4-chlorobenzyl)piperazin-1-yl]propanethiol dihydrochloride, 0.9 g of triethylamine and 50 ml of methylene chloride, to give 0.9 g (20% of theory) of N-(2-methoxy-phenyl)-S-{3-[4-(4-chlorobenzyl)-piperazin-1-yl]propyl}thiocarbamate dihydrochloride sesquihydrate as an off-white crystalline solid, M.p. 238—240°C.

Example 13

N-(3-Methoxyphenyl)-S-{3-[4-(4-chlorobenzyl)piperazin-1-yl]propyl}thiocarbamate dihydrochloride

The procedure described in Example 2 was followed, using 1.3 g of 3-methoxyphenyl isocyanate, 3.0 g of 3-[4-(4-chlorobenzyl)piperazine-1-yl]propanethiol dihydrochloride 0.9 g of triethylamine and 50 ml of methylene chloride, to give 1.35 g (32% of theory) of N-(3-methoxyphenyl)-S-{3-[4-(4-chlorobenzyl)-piperazin-1-yl]propyl}thiocarbamate dihydrochloride as an off-white crystalline solid, M.p. 238—240°C.

Example 14

N-(4-Methoxyphenyl)-S-{3-[4-(4-chlorobenzyl)piperazin-1-yl]propyl}thiocarbamate dihydrochloride monohydrate

The procedure described in Example 2 was followed, using 0.9 g of 4-methoxphenyl isocyanate, 2.0 g of 3-[4-(4-chlorobenzyl)piperazin-1-yl]propanethiol dihydrochloride, 0.7 g of triethylamine and 50 ml of methylene chloride, to give 0.72 g (25% of theory) of N-(4-methoxyphenyl)-S-{3-[4-(4-chlorobenzyl)-piperazin-1-yl]propyl}thiocarbamate dihydrochloride monohydrate as an off-white crystalline solid, M.p. 235—237°C.

Example 15

N-(4-Acetylphenyl)-S-{3-[4-(4-chlorobenzyl)piperazin-1-yl]propyl}thiocarbamate dihydrochloride monohydrate

The procedure described in Example 2 was followed, using 1.0 g of 4-acetylphenyl isocyanate, 2.0 g of 3-[4-(4-chlorobenzyl)piperazin-1-yl]propanethiol dihydrochloride, 0.7 g of triethylamine and 50 ml of methylene chloride, to give 0.83 g (29% of theory) of N-(4-acetylphenyl)-S-{3-[4-(4-chlorobenzyl)piperazin-1-yl]propyl}thiocarbamate dihydrochloride monohydrate as a white crystalline solid, M.p. 235—237°C.

Example 16

N-(4-Ethoxycarbonylphenyl)-S-{3-[4-(4-chlorobenzyl)piperazin-1-yl]propyl}thiocarbamate dihydrochloride

The procedure described in Example 2 was followed, using 1.1 g of 4-ethoxycarbonylphenyl isocyanate, 2.0 g of 3-[4-(4-chlorobenzyl)piperazin-1-yl]propanethiol dihydrochloride, 0.7 g of triethylamine and 50 ml of methylene chloride, to give 0.85 g (28% of theory) of N-(4-ethoxy-carbonylphenyl)-S-{3-[4-(4-chlorobenzyl)piperazin-1-yl]propyl}thiocarbamate dihydrochloride as a white crystalline solid, M.p. 231—233°C.

Example 17

3-[4-(4-Methylbenzyl)piperazin-1-yl)propanethiol dihydrochloride

(a) A mixture of 14.0 g of 1-(4-methylbenzyl)piperazine, 8.0 g of potassium hydroxide and 125 ml of dimethyl sulfoxide was stirred for 45 minutes. 11.8 g of 1-bromo-3-chloropropane were then added, and the mixture was stirred for an additional hour. Water was added, and the product was extracted with ether, and the extract was dried (sodium sulfate) and treated with etheral hydrochloric acid to give 23.3 g (94% of theory) of 1-(4-methylbenzyl)-4-(3-chloropropyl)-piperazine dihydrochloride as a white crystallaine solid.

(b) The procedure described in Example 1(b) was followed, using 7.7 g of thiourea, 23.0 g of 1-(4-methylbenzyl-4-(3-chloropropyl)piperazine dihydrochloride, 6.5 g of triethylamine and 250 ml of reagent ethanol. The hydrolysis was effected with 6.0 g of sodium hydroxide in 40 ml of water. Work-up, as described above, gave 16.4 g (72% of theory) of 3-[4-(4-methylbenzyl)-piperazin-1-yl)propanethiol dihydrochloride as a white crystalline solid, M.p. 273—277°C (dec.).

Example 18

N-Phenyl-S-{3-[4-(4-methylbenzyl)piperazin-1-yl]-propyl}thiocarbamate dihydrochloride sesquihydrate

The procedure described in Example 2 was followed, using 1.4 g of phenyl isocyanate, 4.0 g of 3-[4-(4-methylbenzyl)piperazine-1-yl)propanethiol dihydrochloride, 1.2 g of triethylamine and 50 ml of methylene chloride, to give 1.1 g (20% of theory) of N-phenyl-S-{3-[4-(4-methylbenzyl)piperazin-1-yl]propyl}thiocarbamate dihydrochloride sesquihydrate as a white crystalline solid, M.p. 255—258°C.

Example 19

3-[4-(4-Chlorobenzyl)-2,5-dimethylpiperazin-1-yl]propanethiol dihydrochloride

(a) 22.0 g of 1-bromo-3-chloropropane were added to a mixture of 33.0 g of 4-chlorobenzyl-2,5-dimethylpiperazine, 25.0 g of potassium hydroxide and 125 ml of dimethyl sulfoxide. After stirring the solution for 3 hours at room temperature, water was added, the product was extracted with ether, and the extract was dried (magnesium sulfate) and concentrated to give 25.8 (58% of theory) of 1-(4-chlorobenzyl)-2,5-dimethyl-4-(3-chloropropyl)piperazine as an oil, suitable for use in the next reaction.

(b) The procedure described in Example 1(b) was followed, using 12.7 g of thiourea and 26.0 g of 1-(4-chlorobenzyl)-2,5-dimethyl-4-(3-chloropropyl)piperazine in 250 ml of reagent ethanol. The hydrolysis was effected with 10.0 g of sodium hydroxide in 50 ml of water. Work-up, as described above, gave 26.5 g (83% of theory) of 3-[4-(4-chlorobenzyl)-2,5-dimethylpiperazin-1-yl]-propanethiol dihydrochloride as a white crystalline solid, M.p. 168—171°C.

Example 20

N-Phenyl-S-{3-[4-(4-chlorobenzyl)-2,5-dimethylpiperazin-1-yl}thiocarbamate dihydrochloride

The procedure described in Example 2 was followed, using 1.2 g of phenyl isocyanate, 4.0 g of 1-(4-chlorobenzyl)-2,5-dimethyl-4-(3-chloropropyl)piperazine dihydrochloride, 2.0 g of triethylamine and 50 ml of methylene chloride, to give 1.1 g (22% of theory) of N-phenyl-S-{3-[4-(4-chlorobenzyl)-2,5-dimethylpiperazin-1-yl]propyl}thiocarbamate dihydrochloride as a white crystalline solid, M.p. 215—218°C.

Example 21

3-[4-(1-Phenyl)piperazin-1-yl]propanethiol dihydrochloride

(a) The procedure described in Example 17(a) was followed, using 16.5 g of 1-(1-phenyethyl) piperazine, 8.0 g of potassium hydroxide, 125 ml of dimethyl sulfoxide and 9.5 g of 1-bromo-3-chloropropane. Work-up, as described above, gave 28.0 g (96% of theory) of 1-(1-phenylethyl)-4-(3-chloropropyl)-piperazine dihydrochloride as a white crystalline solid.

(b) The procedure described in Example 1(b) was followed, using 9.8 g of thiourea, 29.0 g of 1-(1-phenylethyl)-4-(3-chloropropyl)piperazine dihydrochloride, 8.3 g of triethylamine and 250 ml of reagent ethanol. The hydrolysis was effected with 6.0 g of sodium hydroxide in 40 ml of water. Work-up, as above, gave 14.5 g (51% of theory) of 3-[4-(1-phenylethyl)piperazin-1-yl]-propanethiol dihydrochloride as a white crystalline solid, M.p. 252—255°C.

Example 22

N-Phenyl-S-{3-[4-(1-phenylethyl)piperazin-1-yl]propyl}thiocarbamate dihydrochloride dihydrate

The procedure described in Example 2 was followed, using 1.6 g of phenyl isocyanate, 4.5 g of 3-[4-(1-phenylethyl)piperazin-1-yl]propanethiol dihydrochloride, 1.3 g of triethylamine and 50 ml of methylene chloride, to give 2.1 g (38% of theory) of N-phenyl-S-{3-[4-(1-phenylethyl)piperazin-1-yl]propyl}thiocarbamate dihydrochloride dihydrate as a white crystalline solid, M.p. 227—229°C.

Example 23

3-(4-Phenyethylpiperazin-1-yl)propanethiol dihydrochloride

(a) The procedure described in Example 1(a) was followed using 29.4 g of 1-phenethyl piperazine, 25.0 g of potassium hydroxide, 100 ml of dimethyl sulfoxide and 24.0 g of 1-bromo-3-chloropropane. Work-up, as described above, gave 32.6 g (64% of theory) of 1-phenethyl-4-(3-chloropropyl)piperazine dihydrochloride as an off-white crystalline solid.

(b) The procedure described in Example 1(b) was followed using 15.2 g of thiourea, 32.6 g of 1-phenethyl-4-(3-chloropropyl)piperazine dihydrochloride, 20.0 g triethylamine and 250 ml of reagent ethanol. The hydrolysis was effected with 10.0 g of sodium hydroxide in 40 ml of water. Work-up, as described above, gave 26.4 g (79% theory) of 3-(4-phenethylpiperazin-1-yl)-propanethiol dihydrochloride as an off-white crystalline solid, M.p. 269—272°C.

Example 24

N-Phenyl-S-[3-(4-phenethylpiperazin-1-yl)propyl]-thiocarbamate dihydrochloride monohydrate

The procedure described in Example 2 was followed, using 1.4 g of phenyl isocyanate, 4.0 g of 3-(4-phenethylpiperazin-1-yl)propanethiol dihydrochloride, 2.4 g of triethylamine and 50 ml of methylene chloride, to give 1.9 g (35% of theory) of N-phenyl-S-[3-(4-phenethylpiperazin-1-yl)-propyl]-thiocarbamate dihydrochloride monohydrate as a white crystalline solid, M.p. 272—275°C.

Example 25

3-[4-(4-Chlorophenethyl)piperazin-1-yl]propanethiol dihydrochloride

(a) The procedure described in Example 17(a) was followed, using 6.5 g of 1-(4-chlorophenethyl) piperazine, 6.0 g of potassium hydroxide, 125 ml of dimnethyl sulfoxide and 4.6 g of 1-bromo-3-chloropropane. Work-up, as described above, gave 6.4 g (59% of theory) to 1-(4-chlorophenethyl)-4-(3-chloropropyl)piperazine dihydrochloride as a white crystalline solid.

(b) The procedure described in Example 1(b) was followed, using 1.3 g of thiourea, 6.4 g of 1-(4-chlorophenethyl)-4-(3-chloropropyl)piperazine dihydrochloride, 1.6 g of triethylamine and 150 ml of reagent ethanol. The hydrolysis was effected with 3.5 g of sodium hydroxide and 40 ml of water. Work-up, as described above, gave 5.6 g (89% of theory) of 3-[4-(4-chlorophenethyl)piperazin-1-yl]propanethiol dihydrochloride as a white crystalline solid, M.p. 279—281°C.

Example 26

N-Phenyl-S-{3-[4-(4-chlorophenethyl)piperazin-1-yl]propyl}thiocarbamate dihydrochloride monohydrate

The procedure described in Example 2 was followed, using 1.6 g of phenyl isocyanate, 5.0 g of 3-[4-(4-chlorophenethyl)piperazin-1-yl]propanethiol dihydrochloride, 1.3 g of triethylamine and 50 ml of methylene chloride, to give 3.0 g (44% of theory) of N-phenyl-S-{3-[4-(4-chlorophenethyl)piperazin-1-yl]propyl}thiocarbamate dihydrochloride monohydrate as a white crystalline solid, M.p. 237—239°C.

Example 27

N-(4-n-Butylphenyl)-S-{3-[4-(4-chlorophenethyl)-piperazin-1-yl]propyl}thiocarbamate dihydrochloride

The procedure described in Example 2 was followed, using 1.4 g of 4-n-butylphenyl isocyanate, 3.0 g of 3-[4-(4-chlorophenethyl)piperazin-1-yl]propanethiol dihydrochloride, 0.8 g of triethylamine and 50 ml of methylene chloride, to give 1.1 g (25% of theory) of N-(4-n-butylphenyl)-S-{3-[4-(4-chlorophenethyl)-piperazin-1-yl]propyl}thiocarbamate dihydrochloride as a white crystalline solid, M.p. 252—254°C.

Example 28

N-(4-Methoxyphenyl)-S-{3-[4-(4-chlorophenethyl)piperazin-1-yl]propyl}thiocarbamate dihydrochloride

The procedure described in Example 2 was followed, using 1.7 g of 4-methoxyphenyl isocyanate, 4.0 g of 3-[4-(4-chlorophenethyl)piperazin-1-yl]propanethiol dihydrochloride, 1.1 g of triethylamine and 50 ml of methylene chloride, to give 1.4 g (25% of theory) of N-(4-methoxyphenyl)-S-{3-[4-(4-chlorophenethyl)-piperazin-1-yl]propyl}thiocarbamate dihydrochloride as a white crystalline solid, M.p. 236—238°C.

Example 29

N-(3,4,5-Trimethoxyphenyl)-S-{3-[4-(4-chlorophenethyl)piperazin-1-yl]propyl}thiocarbamate dihydrochloride

The procedure described in Example 2 was followed, uding 2.5 g of 3,4,5-trimethoxyphenyl isocyanate, 4.4 g of 3-[4-(4-chlorophenethyl)piperazin-1-yl]propanethiol dihydrochloride, 1.2 g of triethylamine and 50 ml of methylene chloride, to give 1.6 g (23% of theory) of N-(3,4,5-trimethoxyphenyl)-S-}3-[4-(4-chlorophenethyl)piperazin-1-yl]propyl}thiocarbamate dihydrochloride as a white crystalline solid, M.p. 239—241°C.

Example 30

3-[4-(3-Phenylpropyl)piperazin-1-yl]propanethiol dihydrochloride

(a) The procedure described in Example 17(a) was followed, using 36.0 g of (3-phenylpropyl)-piperazine, 25.0 g of potassium hydroxide, 125 ml of dimethyl sulfoxide and 28.0 g of 1-bromo-3-chloropropane, to give 46.0 g (73% of theory) of 1-(3-phenylpropyl)-4-(3-chloropropyl)piperazine dihydrochloride.

(b) The procedure described in Example 1(b) was followed using 19.0 g of thiourea, 44.0 g of 1-(3-phenylpropyl)-4-(3-chloropropyl)piperazine dihydrochloride, 25 g of triethylamine and 250 ml of reagent ethanol. The hydrolysis was effected with 10.0 g of sodium hydroxide in 50 ml of water. Work-up, as described above, gave 35.8 g (82% of theory) of 3-[4-(3-phenylpropyl)piperazin-1-yl]propanethiol dihydrochloride as a white crystalline solid, M.p. 237—239°C.

## Example 31

N-Phenyl-S-{3-[4-(3-phenylpropyl)piperazin-1-yl]propyl}thiocarbamate dihydrochloride monohydrate

The procedure described in Example 2 was followed, using 1.2 g of phenyl isocyanate, 4.0 g of 3-[4-(3-phenylpropyl)piperazin-1-yl]propanethiol dihydrochloride, 2.2 g of triethylamine and 50 ml of methylene chloride, to give 2.1 g (41% of theory) of N-phenyl-S-{3-[4-(3-phenylpropyl)piperazin-1-yl]propyl}thiocarbamate dihydrochloride monohydrate as a white crystalline solid, M.p. 240—243°C.

## Example 32

3-{4-[3-(4-Chlorophenyl)propyl]piperazine-1-yl}propanethiol dihydrochloride

(a) The procedure described in Example 17(a) was followed, using 27.0 g of 3-(4-chlorophenyl)propyl piperazine, 22.0 g of potassium hydroxide, 250 ml of diemthyl sulfoxide and 17.7 g of 1-bromo-3-chloropropane to give 22.9 g (54% of theory) of 1-[3-(4-chlorophenyl)propyl]-4-(3-chloropropyl)piperazine dihydrochloride.

(b) The procedure described in Example 1(b) was followed, using 7.6 g of thiourea, 22.9 g of 1-[3-(4-chlorophenyl)propyl]-4-(3-chlorophenyl)piperazine dihydrochloride. 5.8 g of triethylamine and 250 ml of reagent ethanol. The hydrolysis was effected with 10.0 g of sodium hydroxide in 50 ml of water. Work-up, as described above, gave 14.1 g (65% of theory) of 3-{4-[3-(4-chlorophenyl)propyl]piperazin-1-yl}propanethiol dihydrochloride as a white crystalline solid, M.p. 238—252°C.

## Example 33

N-(4-n-Butylphenyl)-S-{3-[4-[3-(4-chlorophenyl)propyl]piperazin-1-yl]propyl}thiocarbamate dihydrochloride

The procedure described in Example 2 was followed, using 2.5 g of 4-n-butylpheny isocyanate, 5.0 g of 3- 4-[3-(4-chlorophenyl)propyl]piperazin-1-yl propanethiol dihydrochloride, to give 2.7 g (34% of theory) of N-(4-n-butylphenyl-S-{3-[4-[3-(4-chlorophenyl)propyl}piperazin-1-yl]propyl}thiocarbamate dihydrochloride as a white crystalline solid, M.p. 270—272°C.

## Example 34

3-[4-(4-Fluorophenyl)piperazin-1-yl]propanethiol dihydrochloride

(a) The procedure described in Example 1(a) was followed, using 32.8 g of 1-(4-fluorobenzyl)piperazine, 30.0 g of potassium hydroxide, 100 ml of dimethyl sulfoxide and 27.0 g of 1-bromo-3-chloropropane. Work-up, as described above, gave 35.2 g (61% of theory) of 1-(4-fluorobenzyl)-4-(3-chloropropyl)piperazine dihydrochloride as a white crystalline solid.

(b) The procedure described in Example 1(b) was followed, using 15.2 g of thiourea, 35.2 g of 1-(4-fluorobenzyl)-4-(3-chloropropyl)piperazine dihydrochloride, 20.0 g of triethylamine and 200 ml of reagent ethanol. The hydrolysis was effected with 10.0 g of sodium hydroxide and 50 ml of water. Work-up, as described above, gave 15.5 g (58% of theory) of 3-[4-(4-fluorophenyl)piperazin-1-yl]propanethiol. The salt was precipitated with ethereal hydrochloric acid to give 3-[4-(4-fluorophenyl)piperazin-1-yl]propanethiol dihydrochloride as a white crystalline solid, m.p. 284—287°C (dec.).

## Example 35

N-Phenyl-S-{3-[4-(4-fluorophenyl)piperazin-1-yl]propyl}thiocarbamate dihydrochloride

1.2 g of phenyl isocyanate was added to a solution of 2.5 g of 3-[4-(4-fluorophenyl)piperazin-1-yl]propanethiol in 75 ml of methylene chloride and the mixture was refluxed for four hours. After the further addition of 100 ml of methylene chloride the solution was washed with water, dried (sodium sulfate) and concentrated. The salt was precipitated with etheral hydrochloric acid and recrystallized from aqueous ethanol to give 1.35 g (33% of theory) of N-phenyl-S-{3-[4-(4-fluorophenyl)piperazin-1-yl]propyl}thiocarbamate dihydrochloride as a white crystalline solid, m.p. 250—252°C.

## Example 36

2-[4-(4-Chlorobenzyl)piperazin-1-yl]ethanethiol dihydrochloride

(a) A mixture of 15.0 g of 1-(4-chlorobenzyl)piperazine, 14.4 g of 1-bromo-2-chlorethane and 150 ml of tetrahydrofuran was refluxed for 8 hours. The solvent was removed in vacuo, and 2N sodium hydroxide was added to the residue. The product was extracted with methylene chloride, and the extract was dried (sodium sulfate) and concentrated. The salt was precipitated with etheral hydrochloric acid to give 13.8 g (56% of theory) of 1-(4-chlorobenzyl)-4-(2-chloroethyl)piperazine dihydrochloride as a white crystalline solid.

(b) The procedure described in Example 1(b) was followed, using 6.1 g of thiourea, 13.8 g of 1-(4-chlorobenzyl)-4-(2-chloroethyl)piperazine dihydrochloride, 4.1 g of triethylamine and 150 ml of reagent ethanol. The hydrolysis was effected with 7.0 g of sodium hydroxide and 50 ml of water. Work-up, as described above, gave 6.7 g (62% of theory) of 2-[4-(4-chlorobenzyl)piperazin-1-yl]ethanethiol dihydrochloride as a white crystalline solid, m.p. 244—247°C.

Example 37

N-Phenyl-S-{2-[4-(4-chlorobenzyl)piperazin-1-yl]ethyl}thiocarbamate dihydrochloride

The procedure described in Example 35 was followed, using 1.3 g of phenyl isocyanate, 3.0 g of 2-[4-(4-chlorobenzyl)piperazin-1-yl]ethanethiol and 100 ml of methylene chloride, to give 2.2 g (43% of theory) of N-phenyl-S-{2-[4-(4-chlorobenzyl)piperazin-1-yl]ethyl}thiocarbamate dihydrochloride as a white crystalline solid, m.p. 232—236°C (dec.).

Example 38

N-Cyclohexyl-S-{3-[4-(4-chlorophenethyl)piperazin-1-yl]propyl}thiocarbamate dihydrochloride

The procedure described in Example 35 was followed, using 1.7 g of cyclohexyl isocyanate, 4.0 g of 3-[4-(4-chlorophenethyl)piperazin-1-yl]propanethiol and 100 ml of methylene chloride, to give 3.8 g (58% of theory) of N-cyclohexyl-S-{3-[4-(4-chlorophenethyl)piperazin-1-yl]propyl}thiocarbamate dihydrochloride as a white crystalline solid, m.p. 262—267°C.

Example 39

N-(4-Methoxyphenyl)-S-{3-[4-[3-(4-chlorophenyl)propyl]piperazin-1-yl]propyl}thiocarbamate dihydrochloride hemihydrate

The procedure described in Example 35 was followed, using 2.0 g of 4-methoxyphenyl isocyanate, 4.0 g of 3-{4-[3-(4-chlorophenyl)propyl]piperazin-1-yl}propanethiol and 100 ml of methylene chloride to give 3.4 g (48% of theory) of N-(4-methoxyphenyl)-S-{3-[4-[3-(4-chlorophenyl)propyl]piperazin-1-yl]propyl}thiocarbamate dihydrochloride hemihydrate as a white crystalline solid, m.p. 240—244°C.

Example 40

N-(n-Hexyl)-S-{3-[4-(4-chlorobenzyl)piperazin-1-yl]propyl}thiocarbamate dihydrochloride

The procedure described in Example 35 was followed, using 1.4 g of n-hexyl isocyanate, 3.0 g of 3-[4-(4-chlorobenzyl)piperazin-1-yl]propanethiol and 100 ml of methylene chloride, to give 2.7 g (51% of theory) of N-(n-hexyl)-S-{3-[4-(4-chlorobenzyl)piperazin-1-yl]propyl}thiocarbamate dihydrochloride as a white crystalline solid, m.p. 249—252°C (dec.).

Example 41

3-{4-[4-(4-Chlorophenyl)butyl]piperazin-1-yl}propanethiol dihydrochloride

(a) The procedure described in Example 1(a) was followed, using 10.0 g of 4-(4-chlorophenyl)butyl piperazine, 47.2 g of 1-bromo-3-chloropropane, 150 ml of dimethyl sulfoxide and 11.2 g of potassium hydroxide, to give 10.0 g (82% of theory) of 1-[4-(4-chlorophenyl)butyl]-4-(3-chloropropyl)piperazine dihydrochloride as a white crystalline solid, m.p. 266—268°C.

(b) The procedure described in Example 1(b) was followed, using 1.0 g of thiourea, 4.0 g of 1-[4-(4-chlorophenyl)butyl]-4-(3-chloropropyl)piperazine dihydrochloride and 50 ml of reagent ethanol. The hydrolysis was effected with 4.0 g of sodium hydroxide in 50 ml of water. Work-up of the free base, as described above, followed by purification on a silica gel column (methylene chloride:methanol, 97:3), precipitation as the hydrochloride salt and recrystallization from reagent ethanol, gave 0.6 g (16% of theory) of 3-{4-[4-(4-chlorophenyl)butyl]piperazin-1-yl}propanethiol dihydrochloride as a white crystalline solid, m.p. 254—259°C.

Example 42

N-(n-Hexyl)-S-{3-[4-[4-(4-chlorophenyl)butyl]piperazin-1-yl]propyl}thiocarbamate dihydrochloride

The procedure described in Example 2 was followed, using 1.0 g of n-hexyl isocyanate, 2.6 g of 3-{4-[4-(4-chlorophenyl)butyl]piperazin-1-yl}propanethiol and 25 ml of methylene chloride, to give 1.4 g (32% of theory) of N-(n-hexyl)-S-{3-[4-(4-chlorophenyl)butyl]piperazin-1-yl]propyl}thiocarbamate dihydrochloride as a white crystalline solid, m.p 235—245°C (dec.).

Example 43

3-(4-Cinnamylpiperazin-1-yl)propanethiol dihydrochloride

(a) 6.0 g of cinnamyl bromide were added to a mixture of 7.4 g of 1-(3-chloropropyl)piperazine dihydrochloride hemihydrate, 6.1 g of triethylamine and 100 ml of reagent ethanol. The mixture was refluxed for 4 hours and then stirred overnight. After removal of the solvent in vacuo, water was added to the residue. The product was then extracted with ether and the extract was dried (magnesium sulfate) and concentrated. The resulting oil was chromatographed on silica gel (methylene chloride/methanol: 9/1) and precipitated with etheral hydrochloric acid to give 7.4 g (70% of theory) of 1-cinnamyl-4-(3-chloropropyl)piperazine dihydrochloride.

(b) The procedure described in Example 1(b) was followed, using 3.1 g of thiourea, 7.4 g of 1-cinnamyl-4-(3-chloropropyl)piperazine dihydrochloride, 4.1 g of triethylamine and 100 ml of reagent ethanol. The hydrolysis was effected with 3.0 g of sodium hydroxide in 30 ml of water. Work-up, as described above, gave 3.6 g (52% of theory) of 3-(4-cinnamylpiperazin-1-yl)propanethiol dihydrochloride as a white crystalline solid, m.p. 249—252°C.

| Compounds | Mediator Release Inhibition $IC_{50}$ (μM)* RPMC | GPBL | HBL |
|---|---|---|---|
| N-Cyclohexyl-S-{3-[4-(4-chlorobenzyl)piperazin-1-yl]propyl}thiocarbamate dihydrochloride | 3 | 10 | 3 |
| 3-[4-(4-Chlorophenethyl)-piperazin-1-yl]propanethiol dihydrochloride | 6 | 5 | 5 |
| N-(4-Methoxyphenyl)-S-{3-[4-(4-chlorophenethyl)piperazin-1-yl]propyl}thiocarbamate dihydrochloride | 6 | 40 | 3 |
| N-(4-n-Butylphenyl)-S-}3-[4-[3-(4-chlorophenyl)propyl]-piperazin-1-yl]propyl}-thiocarbamate dihydrochloride | 5 | 10 | 4 |
| Theophylline | >1000 | 200 | 400 |
| DSCG | | | >1000 |

* Concentration of the compounds required in the cellular reaction mixture to inhibit 50% of the release of the pharmacological mediators from the target cells.

[1] Y. Morita and R. P. Siraganian, J. Immunol. 127, 1339 (1981).

[2] M. A. Lett-Brown, D. O. Thueson and J. A. Grant, Int. Arch. Allergy Appl. Immunol. 64, 241 (1981)

[3] R. P. Siraganian and W. Hook in "Manual of Clinical Immunology", 2nd Edition, N. R. Rose and H. Friedman, ed., pp. 808, Washington, D.C. (1980).

For pharmaceutical purposes the compounds of the present invention are administered to warm-blooded animals topically, perorally, parenterally or by the respiratory route as active ingredients in customary pharmaceutical compositions, that is, compositions consisting essentially of an inert pharmaceutical carrier and an effective amount of the active ingredient. The oral and the topical route are preferred.

When they are to be administered by the oral route the compounds of the present invention may be compounded in the form of syrups, tablets, capsules, pills and the like. Preferably, the compositions are in unit dosage form, or in a form in which the patient can administer to himself a single dose. When the composition is in the form of a tablet, powder or lozenge, any pharmaceutical carrier suitable for formulating solid compositions may be used. Examples of such carriers are various starches, lactose, glucose, sucrose, cellulose, dicalcium phosphate, and chalk. The composition may also be in the form of an ingestible capsule (for example, of gelatin) containing the compound; or in the form of a syrup, a liquid solution or a suspension. Suitable liquid pharmaceutical carriers include ethyl alcohol, glycerin, saline, water, propylene glycol, sorbitol solution which may be compounded with flavoring or coloring agents to form syrups.

The compounds of this invention may also be administered by other than the oral route. In accordance with routine pharmaceutical procedure, the composition may be formulated, for example, for rectal administration as a suppository or for presentation in an injectable form in an aqueous or non-aqueous solution, suspension or emulsion in a pharmaceutically acceptable liquid, such as sterile pyrogen-free water or a parenterally acceptable oil or a mixture of liquids, which may contain bacteriostatic agents, antioxidants, preservatives, buffers, or other solutes to render the solution isotonic with the blood, thickening agents, suspending agents or other pharmaceutically acceptable additives. Such forms will be presented in unit dose forms such as ampules or disposable injection devices, or in multi-dose vials such as a bottle from which the appropriate dose may be withdrawn, or as a solid form or concentrate which can be used to prepare an injectable formulation.

The compounds of this invention may also suitably be presented for administration via the respiratory tract as an aerosol or solution for a nebulizer, or as a microfine powder for insufflation, alone or in combination with an inert carrier such as lactose. In such a case the particles of active compounds suitably have diameters of less than 20 microns, preferably less than 10 microns. Where appropriate, small amounts of other antiallergics, anti-asthmatic and bronchodilators for example sympathomimetic amines such as isoprenaline, isoetharine, metaproterenol, salbutamol, phenylephrine, fenoterol, and ephedrine; xanthine derivatives such as theophylline and aminophylline; and corticosteroids such as prednisolone and adrenal stimulants such as ACTH may be included.

The compounds of this invention may also be presented as an ointment, cream, lotion, gel, aerosol, or solution for topical application to the skin, nose, or eye.

Topical solution for the nose and the eye may contain, in addition to the compounds of this invention, suitable buffers, toxicity adjusters, microbial preservatives, antioxidants and viscosity-impairing agents in an aqueous vehicle. Examples of agents used to increase viscosity are polyvinyl alcohol, cellulose derivatives, polyvinylpyrrolidone, polysorbates or glycerin. Microbial preservatives added may include benzalkonium chloride, thimerosal, chlorobutanol or phenylethyl alcohol. Topical preparations for the eye may also be presented as ointments in a suitable inert base consisting of mineral oil, petrolatum, polyethylene glycols or lanolin derivatives, along with microbial preservatives.

In addition to these dosage forms, a skin paint can also be formulated for application to the skin.

In any of the foregoing formulations, a suitable dosage unit may contain from 0.005 to 500 mg of the active ingredient. The effective dose of a compound of this invention depends on the particular compound employed, the condition of the patient, and on the frequency and route of administration, but in general is in the range of from 0.0001 mg/kg to 10 mg/kg, inclusive, of the patient's body weight.

In the preferred topical administration, a 0.001 to 0.5%, preferably a 0.01 to 0.1% solution is provided for. As is common practice, the composition will usually be accompanied by written or printed directions for use in the medical treatment concerned, in this case as an anti-allergic agent for the prophylaxis and treatment of for example, asthma, hayfever, rhinitis or allergic eczema.

The following example illustrates the preparation and properties of compositions of this invention. For the preparation of pharmaceutical compositions, the compounds of the formula I are mixed in the usual way with appropriate pharmaceutical carrier substances and aroma, flavoring and coloring materials and formed, for example, into tablets or capsules or, with the addition of appropriate adjuvants, suspended or dissolved in water or in an oil, for example corn oil.

The compounds of the formula I can be administered orally and parenterally in liquid or solid form. As injection medium, it is preferred to use water which contains the stabilizing agents, solubilizing agents and/or buffers conventionally used for injection solutions. Additives of this type include, for example, tartrate, citrate and acetate buffers, ethanol, propylene glycol, polyethylene glycol, complex formers (such as EDTA), antioxidants (such as sodium bisulfite, sodium metabisulfite or ascorbic acid), high molecular weight polymers (such as liquid polyethylene oxides) for viscosity regulation, and polyethylene derivatives of sorbitol anhydrides.

Preservatives may also be added if necessary, such as benzoic acid, methyl or propyl paraben, benzalkonium chloride and other quaternary ammonium compounds. Solid carrier material which can be used include, for example, starch, lactose, mannitol, methyl cellulose, microcrystalline cellulose, talc, silica, dibasic calcium phosphate, and high molecular weight polymers (such as polyethylene glycol).

Compositions suitable for oral administration can, if desired, contain flavoring and/or sweetening agents. For topical administration, the compounds of the present invention can also be used in the form of powders or ointments, for which purpose they are mixed with, for example, powdered, physiologically compatible diluents or conventional ointment bases.

The following examples illustrate a few pharmaceutical dosage unit compositions comprising a compound of the present invention as an active ingredient and represent the best modes contemplated of using the invention. The parts are parts by weight unless otherwise specified.

## Example 48

*Tablets*

The tablet composition is compounded from the following ingredients:

| | | |
|---|---|---|
| N-Phenyl-S-{3-[4-(4-chlorobenzyl)-piperazin-1-yl]propyl}thio-carbamate dihydrochloride | | 0.010 parts |
| Stearic acid | | 0.010 parts |
| Dextrose | | 1.890 parts |
| | Total | 1.910 parts |

Preparation:

The ingredients are admixed in conventional manner, and the mixture is compressed into 1.91 gm-tablets, each of which is an oral dosage unit composition containing 10 mg of the active ingredient.

Example 44

N-(n-Hexyl)-S-[3-(4-cinnamylpiperazin-1-yl)propyl]thiocarbamate dihydrochloride

The procedure described in Example 2 was followed, using 0.4 g of n-hexyl isocyanate, 1.0 g of 3-(4-cinnamyl-piperazin-1-yl)propanethiol dihydrochloride, 0.6 g of triethylamine and 50 ml of methylene chloride, to give 0.44 g (31% of theory) of N-(n-hexyl)-S-[3-(4-cinnamylpiperazin-1-yl]propyl]thiocarbamate dihydrochloride as a white crystalline solid, m.p. 251—253°C.

Example 45

3-[4-(4-Chlorobenzyl)homopiperazin-1-yl]propanethiol

(a) 15.7 g of 1-bromo-3-chloropropane were added to a cooled solution of 15.0 g of 1-(4-chlorobenzyl)homopiperazine and 12.0 g of potassium hydroxide in 50 ml of dimethyl sulfoxide. The mixture was stirred at 5—10°C for one hour, and then an additional 30 minutes at room temperature. After the addition of ice water, the product was extracted with ether, and the extract was dried (magnesium sulfate) and concentrated in vacuo. Final purification was effected on a silica gel column (methylene chloride:methanol, 9:1), and the solvent was removed in vacuo to give 8.4 g (42% of theory) of 1-(4-chlorobenzyl)-4-(3-chloropropyl)-homopiperazine as an oil.

(b) 4.1 g of thiourea were added to a mixture of 8.0 g of 1-(4-chlorobenzyl)-4-(3-chloropropyl)homopiperazine and 100 ml of reagent ethanol. The mixture was refluxed for 6 hours and then stirred overnight at room temperature. A solution of 5.0 g of sodium hydroxide in 30 ml of water was added, and the resulting solution was refluxed for 4 hours. The ethanol was removed in vacuo, and water was added to the residue. The product was extracted with methylene chloride, and the extract was dried (sodium sulfate) and concentrated to give 3.1 g (39% of theory) of 3-[4-(4-chlorobenzyl)homopiperazin-1-yl]propanethiol as a yellow oil.

Example 46

N-(n-Hexyl)-S-{3-[4-(4-chlorobenzyl)homopiperazin-1-yl]propyl}thiocarbamate dihydrochloride

The procedure described in Example 2 was followed, using 1.3 g of n-hexyl isocyanate, 3.0 g of 3-[4-(4-chlorobenzyl)homopiperazin-1-yl]propanethiol and 75 ml of methylene chloride, to give 1.3 g (33% of theory) of N-(n-hexyl)-S-{3-[4-(4-chlorobenzyl)homopiperazin-1-yl]propyl}thiocarbamate dihydrochloride as a tan powder, m.p. 183-187°C.

Example 47

N-[t-Butyl)-S-{3-]4-(4-chlorobenzyl)piperazin-1-yl]propyl}thiocarbamate dihydrochloride sesquihydrate

The procedure described in Example 2 was followed, using 0.8 g of t-butyl isocyanate, 3.0 g of 3-[4-(4-chlorobenzyl)piperazin-1-yl]propanethiol dihydrochloride, 1.6 g of triethylamine and 75 ml of methylene chloride, to give 2.2 g (60% of theory) of N-(t-butyl)-S-{3-[4-(4-chlorobenzyl)piperazin-1-yl]propyl}thiocarbamate dihydrochloride sesquihydrate as a white crystalline solid, m.p. 246—248°C.

The compounds of the present invention, that is, those embraced by formula I above and their non-toxic, pharmacologically acceptable acid addition salts, have useful pharmacodynamic properties. More particularly, they exhibit potent inhibition of mediator release in numerous cell systems of warm-blooded animals such as rats, guinea pigs and humans, and are therefore useful for the treatment of allergic diseases such as allergic asthma, rhinitis, conjunctivitis, hay fever, urticaria, food allergies and the like.

Moreover, it has been realized that also compounds described in the above-mentioned Japanese Patent No. 6813468 and excluded, therefore, from formula I above, namely compounds wherein n is 2, when R is hydrogen and $R_1$ is 2-methyl, may exhibit similar pharmacological activity. This pharmacological activity, however, is not mentioned in the Japanese patent. The new compounds inhibit, at a concentration of as low as 1 µMol, the release of histamine and other mediators or anaphylaxis from granule containing cells like human basophils and mast cells, measured according to the following method:

Mediator release from mast cells and basophils has been implicated in many allergic and inflammatory disorders. The activity of compounds in inhibiting the non-cytoxic exocytosis of such mediators can be evaluated in in vitro models such as the inhibition of mediator release from isolated cellular systems induced by antigen-antibody interaction.

We include here data obtained in examples of such models to illustrate the biological activity of the novel compounds and the broad scope of their activity. This information is provided in the following table. The cellular systems cited therein are as follows:

RPMC: Rat peritoneal mast cell preparation[1]
GPBL: Guinea pig leukocytes[2]
HBL: Human basophil-enriched leukocytes[3]

A comparison with the activities of the clinical standards theophylline and disodium cromoglycate (DSCG) in these systems is also provided.

Example 49

*Ointment*

The ointment composition is compounded from the following ingredients:

| | | |
|---|---|---|
| 3-[4-(4-Chlorophenethyl)piperazin-1-yl]propanethiol dihydrochloride | | 2.000 parts |
| Fuming hydrochloric acid | | 0.011 parts |
| Sodium pyrosulfite | | 0.050 parts |
| Mixture (1:1) of cetyl alcohol and stearyl alcohol | | 20.000 parts |
| White vaseline | | 5.000 parts |
| Synthetic bergamot oil | | 0.075 parts |
| Distilled water | q.s. ad | 100.000 parts |

Preparation:

The ingredients are uniformly blended in conventional manner into an ointment, 100 gm of which contain 2.0 gm of the active ingredient.

Example 50

*Inhalation aerosol*

The aerosol composition is compounded from the following ingredients:

| | | |
|---|---|---|
| N-Cyclohexyl-S-{3-[4-(4-chlorobenzyl)piperazin-1-yl]propyl}thiocarbamate dihydrochloride | | 1.00 parts |
| Soybean lecithin | | 0.20 parts |
| Propellant gas mixture (Freon 11, 12 and 14) | q.s. ad | 100.00 parts |

Preparation:

The ingredients are compounded in conventional manner and the composition is filled into aerosol containers equipped with a metering valve which releases 0.5 to 2.0 mg of active ingredient per actuation of the valve.

Example 51

*Hypodermic solution*

The solution is compounded from the following ingredients:

| | | |
|---|---|---|
| N-(4-Methoxyphenyl)-S-{3-[4-(4-chlorophenethyl)piperazin-1-yl]propyl}thiocarbamate dihydrochloride | | 5.0 parts |
| Sodium pyrosulfite | | 1.0 parts |
| Sodium salt of EDTA | | 0.5 parts |
| Sodium chloride | | 8.5 parts |
| Double-distilled water | q.s. ad | 1000.0 parts |

Preparation:

The individual ingredients are dissolved in a sufficient amount of double-distilled water, the solution is diluted to the indicated concentration with additional double-distilled water, the resulting solution is filtered until free from suspended particles, and the filtrate is filled under aseptic conditions into 1 ml-ampules which are subsequently sterilized and sealed. Each ampule contains 5 mg of the active ingredient.

Example 52

*Topical solution (ophthalmic or nasal)*

The solution is compounded from the following ingredients:

| | | |
|---|---|---|
| N-(4-n-Butylphenyl)-S-{3-[4-[3-(4-chlorophenyl)propyl]-piperazin-1-yl]propyl}thiocarbamate dihydrochloride | | 0.020 parts |
| Disodium hydrogen phosphate | | 0.758 parts |
| Dihydrogen sodium phosphate | | 0.184 parts |
| Sodium chloride | | 0.365 parts |
| Polyvinyl alcohol | | 3.500 parts |
| Benzalkonium chloride | | 0.010 parts |
| Distilled water | q.s. ad | 100.000 parts |

Preparation:

The ingredients are dissolved in conventional manner to form an aqueous solution. The solution is appropriately filtered, with the ophthalmic solution requiring sterile filtration. Each ml of the solution contains 0.2 mg of the active ingredient.

Any one of the other compounds embraced by formula I or a non-toxic salt thereof may be substituted for the particular active ingredient in examples 48 through 52. Likewise, the amount of active ingredient in these illustrative examples may be varied to achieve the dosage unit range set forth above, and the amounts and nature of the inert pharmaceutical carrier ingreident may be varied to meet particular requirements.

**Claims**

1. A compound of the formula

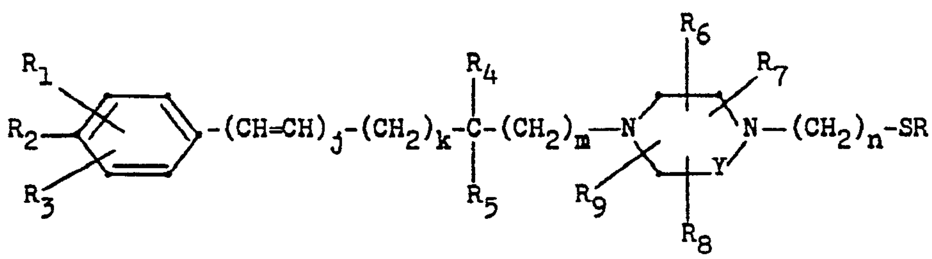

wherein

R is hydrogen or

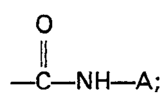

A is alkyl of 1 to 8 carbon atoms; cycloalkyl of 3 to 7 carbon atoms; or unsubstituted or mono-, di- or trisubstituted phenyl, where the substituents are each independently alkyl of 1 to 4 carbon atoms, halogen, trihalomethyl, alkoxy of 1 to 3 carbon atoms, carboxylic acyl of 1 to 3 carbon atoms, carboxyl (alkoxy of 1 to 3 carbon atoms)carbonyl, nitro, cyano or di(alkyl of 1 to 3 carbon atoms)amino;

$R_1$, $R_2$ and $R_3$ are each independently hydrogen, halogen, alkyl of 1 to 4 carbon atoms, trihalomethyl, nitro, cyano, di(alkyl of 1 to 4 carbon atoms)amino, (alkoxy of 1 to 4 carbon atoms) carbonyl, alkoxy of 1 to 4 carbon atoms or hydroxyl;

$R_4$ and $R_5$ are each independently hydrogen, alkyl of 1 to 4 carbon atoms or phenyl;

$R_6$, $R_7$, $R_8$ and $R_9$ are each independently hydrogen or methyl;

Y is $-CH_2-$ or $-CH_2-CH_2-$;

j is 0 or 1;

k and m are each integers of 0 to 3, their sum being no more than 6 and must be 0 when j is 1; and

n is an integer of 2 to 4, with the proviso that n must be 3 or 4 when $R_1$ is hydrogen or 2-methyl, $R_2$, $R_3$, $R_4$, $R_5$, $R_6$, $R_7$, $R_8$, $R_9$ and R are hydrogen and j, k and m are 0 and Y is $-CH_2-$;

or a non-toxic, pharmaceutically acceptable acid addition salt thereof.

2. A compound of claim 1 which is of the formula

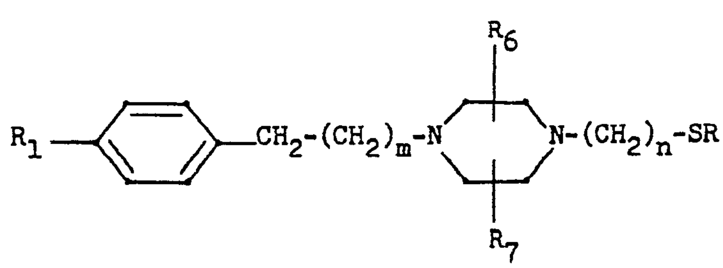

wherein

R is hydrogen or

$$-\overset{\overset{O}{\|}}{C}-NH-A;$$

A is alkyl of 1 to 8 carbon atoms; cycloalkyl of 3 to 7 carbon atoms; or unsubstituted or mono-, di- or trisubstituted phenyl, where the substituents are each independently alkyl of 1 to 4 carbon atoms, halogen, trihalomethyl, alkoxy of 1 to 3 carbon atoms, carboxylic acyl of 1 to 3 carbon atoms, carboxyl, (alkoxy of 1 to 3 carbon atoms)carbonyl, nitro, cyano or di(alkyl of 1 to 3 carbon atoms)amino;

$R_1$ is hydrogen, halogen, alkyl of 1 to 4 carbon atoms, trihalomethyl, nitro, cyano, di(alkyl of 1 to 4 carbon atoms)amino, (alkoxy of 1 to 4 carbon atoms)carbonyl, alkoxy of 1 to 4 carbon atoms or hydroxyl;

$R_6$ and $R_7$ are each independently hydrogen or methyl;

m is an integer from 0 to 3; and

n is an integer of 2 to 4, with the proviso that n must be 3 or 4 when $R_1$ is hydrogen or 2-methyl, $R_6$, $R_7$ and R are hydrogen and m is 0;

and a non-toxic, pharmaceutically acceptable acid addition salt thereof.

3. A compound of claim 2, where

R is hydrogen or

$$-\overset{\overset{O}{\|}}{C}-NH-A;$$

A is alkyl of 1 to 3 carbon atoms; cycloalkyl of 5 to 6 carbon atoms; or unsubstituted or mono-, di- or trisubstituted phenyl, where the substituents are each independently alkyl of 1 to 4 carbon atoms, fluorine, chlorine, bromine, trifluoromethyl, methoxy, acetyl or carbethoxy;

$R_1$ is hydrogen, fluorine, chlorine, bromine, alkyl of 1 to 4 carbon atoms, trifluoromethyl or dimethylamino;

$R_6$ and $R_7$ are each independently hydrogen or methyl;

m is an integer from 0 to 3; and

n is an integer of 2 to 4, with the proviso that n must be 3 or 4 when $R_1$ is hydrogen or 2-methyl, $R_6$, $R_7$ and R are hydrogen and m is 0;

or a non-toxic, pharmaceutically acceptable acid addition salt thereof.

4. The compound of claim 1 which is N-cyclohexyl-S-{3-[4-(4-chlorobenzyl)piperazin-1-yl]propyl}thiocarbamate dihydrochloride.

5. The compound of claim 1 which is 3-[4-(4-chlorophenethyl)piperazin-1-yl]propanethiol dihydrochloride.

6. The compound of claim 1 which is N-(4-methoxyphenyl)-S-{3-[4-(4-chlorophenethyl)piperazin-1-yl]propyl}thiocarbamate dihydrochloride.

7. The compound of claim 1 which is N-(4-n-butylphenyl)-S-{3-[4-[3-(4-chlorophenyl)propyl]piperazin-1-yl]propyl}thiocarbamate dihydrochloride.

8. The compound of claim 1 which is 3-[4-(4-chlorobenzyl)piperazin-1-yl]propanethiol dihydrochloride.

9. The compound of claim 1 which is N-phenyl-S-{3-[4-(4-chlorobenzyl)piperazin-1-yl]propyl}thiocarbamate dihydrochloride.

10. The compound of claim 1 which is 2-[4-(4-chlorobenzyl)piperazin-1-yl]ethanethiol dihydrochloride.

11. The compound of claim 1 which is N-(4-fluorophenyl)-S-{3-[4-(4-chlorobenzyl)piperazin-1-yl]propyl}thiocarbamate dihydrochloride.

12. The compound of claim 1 which is N-(3,4,5-trimethoxyphenyl)-S-{3-[4-(4-chlorophenethyl)piperazin-1-yl]propyl}thiocarbamate dihydrochloride.

13. The compound of claim 1 which is N-phenyl-S-{2-[4-(4-chlorobenzyl)piperazin-1-yl]ethyl}thiocarbamate dihydrochloride.

14. The compound of claim 1 which is N-cyclohexyl-S-{3-[4-(4-chlorophenethyl)piperazin-1-yl]propyl}thiocarbamate dihydrochloride.

15. The compound of claim 1 which is N-(4-methoxyphenyl)-S-{3-[4-[3-(4-chlorophenyl)propyl]piperazin-1-yl]propyl}thiocarbamate dihydrochloride hemihydrate.

16. The compound of claim 1 which is N-(n-hexyl)-S-{3-[4-(4-chlorobenzyl)homopiperazin-1-yl]propyl}thiocarbamate dihydrochloride.

17. An immunological, antiallergic or antiinflammatory composition consisting essentially of an inert pharmaceutical carrier and an effective immunological, antiallergic or antiinflammatory amount of a compound of claim 1.

18. The use of a compound as defined in Claim 1 in the manufacture of a pharmaceutical composition suitable for peroral, parenteral or topical administration for the treatment of immunological, allergic or inflammatory reactions in a warm-blooded animal.

19. The method of preparing a compound of the formula

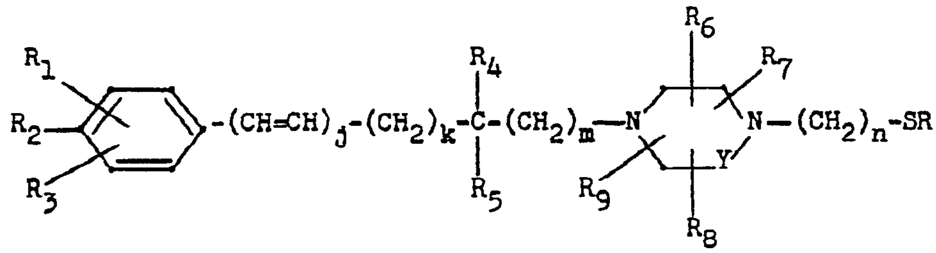

wherein

R is hydrogen or

$$-\overset{\displaystyle O}{\overset{\|}{C}}-NH-A;$$

A is alkyl of 1 to 8 carbon atoms; cycloalkyl of 3 to 7 carbon atoms; or unsubstituted or mono-, di- or trisubstituted phenyl, where the substituents are each independently alkyl of 1 to 4 carbon atoms, halogen, trihalomethyl, alkoxy of 1 to 3 carbon atoms, carboxylic acyl of 1 to 3 carbon atoms, carboxyl, (alkoxy of 1 to 3 carbon atoms)carbonyl, nitro, cyano or di(alkyl of 1 to 3 carbon atoms)amino;

$R_1$, $R_2$ and $R_3$ are each independently hydrogen, halogen, alkyl of 1 to 4 carbon atoms, trihalomethyl, nitro, cyano, di(alkyl of 1 to 4 carbon atoms)amino, (alkoxy of 1 to 4 carbon atoms)carbonyl, alkoxy of 1 to 4 carbon atoms or hydroxyl;

$R_4$ and $R_5$ are each independently hydrogen, alkyl of 1 to 4 carbon atoms or phenyl;

$R_6$, $R_7$, $R_8$ and $R_9$ are each independently hydrogen or methyl;

Y is $-CH_2-$ or $-CH_2-CH_2-$;

j is 0 or 1;

k and m are each integers of 0 to 3, their sum being no more than 6 and must be 0 when j is 1; and

n is an integer of 2 to 4, with the proviso that n must be 3 or 4 when $R_1$ is hydrogen or 2-methyl, $R_2$, $R_3$, $R_4$, $R_5$, $R_6$, $R_7$, $R_8$, $R_9$ and R are hydrogen and j, k and m are 0 and Y is $-CH_2-$;

which comprises reacting a compound of the formula

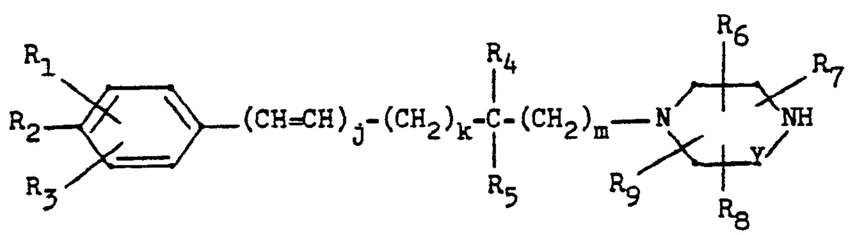

wherein $R_1$, $R_2$, $R_3$, $R_4$, $R_5$, $R_6$, $R_7$, $R_8$, $R_9$, j, k, m and Y have the meanings previously defined, with an alkyl halide of the formula

$$X-(CH_2)_n-Z$$

wherein

X and Z are identical or different halogens, and

n has the meanings previously defined,

reacting the alkyl halide of the formula

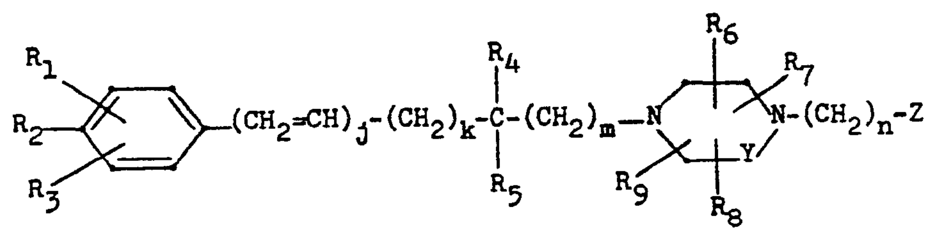

wherein $R_1$, $R_2$, $R_3$, $R_4$, $R_5$, $R_6$, $R_7$, $R_8$, $R_9$, Y, Z, j, k, m and n have the meanings previously defined, thus obtained with thiourea, subjecting the resulting intermediate isothiouronium halide to hydrolysis to obtain an end product of the formula I wherein R is hydrogen, and if desired reacting said end product with an isocyanate of the formula

A—N=C=O

wherein A has the meanings previously defined, to obtain an end product of the formula I wherein R is —CO—NH—A.

**Patentansprüche**

1. Eine Verbindung der Formel

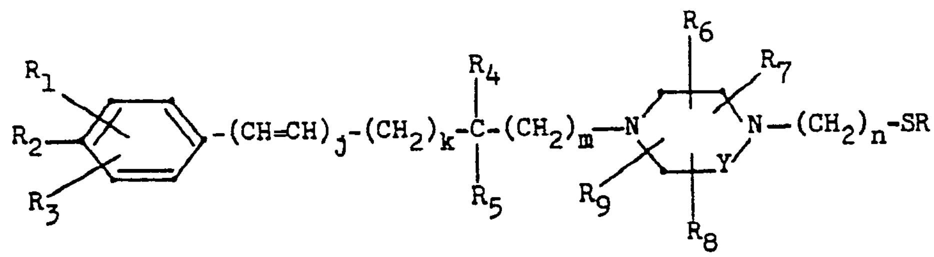

worin

R Wasserstoff oder

O
‖
—C—NH—A ist;

A Alkyl mit 1 bis 8 Kohlenstoffatomen, Cycloalkyl mit 3 bis 7 Kohlenstoffatomen; oder unsubstituiertes oder mono-, di- oder trisubstituiertes Phenyl ist, wobei die Substituenten jeweils unabhängig Alkyl mit 1 bis 4 Kohlenstoffatomen, Halogen, Trihalomethyl, Alkoxy mit 1 bis 3 Kohlenstoffatomen, carboxylisches Acyl mit 1 bis 3 Kohlenstoffatomen, Carboxyl, (Alkoxy mit 1 bis 3 Kohlenstoffatomen)-carbonyl, Nitro, Cyano oder Di(alkyl mit 1 bis 3 Kohlenstoffatomen)-amino sind;

$R_1$, $R_2$ und $R_3$ jeweils unabhängig Wasserstoff, Halogen, Alkyl mit 1 bis 4 Kohlenstoffatomen, Trihalomethyl, Nitro, Cyano, Di-(alkyl mit 1 bis 4 Kohlenstoffatomen)-amino, (Alkoxy mit 1 bis 4 Kohlenstoffatomen)-carbonyl, Alkoxy mit 1 bis 4 Kohlenstoffatomen oder Hydroxyl sind;

$R_4$ und $R_5$ jeweils unabhängig Wasserstoff, Alkyl mit 1 bis 4 Kohlenstoffatomen oder Phenyl sind;

$R_6$, $R_7$, $R_8$ und $R_9$ jeweils unabhängig Wasserstoff oder Methyl sind;

Y —$CH_2$— oder —$CH_2$—$CH_2$— ist;

j 0 oder 1 ist;

k und m jeweils Zahlen von 0 bis 3 sind, wobei ihre Summe nicht mehr als 6 ist und 0 sein muß, wenn j 1 ist; und

n eine Zahl von 2 bis 4 ist, mit der Maßgabe, daß n 3 oder 4 sein muß, wenn $R_1$ Wasserstoff oder 2-Methyl bedeutet, $R_2$, $R_3$, $R_4$, $R_5$, $R_6$, $R_7$, $R_8$, $R_9$ und R Wasserstoff darstellen und j, k und m 0 sind und Y für —$CH_2$— steht;

oder ein nicht-toxisches, pharmazeutisch annehmbares Säureadditionssalz davon.

2. Eine Verbindung gemäß Anspruch 1 der Formel

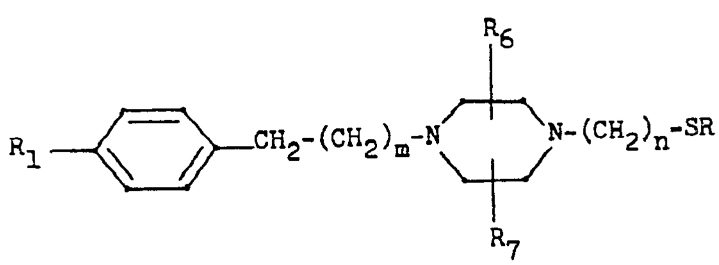

worin

R Wasserstoff oder

$$-\overset{\displaystyle O}{\overset{\|}{C}}-NH-A \text{ ist;}$$

A Alkyl mit 1 bis 8 Kohlenstoffatomen; Cycloalkyl mit 3 bis 7 Kohlenstoffatomen; oder unsubstituiertes oder mono-, di- oder trisubstituiertes Phenyl ist, wobei die Substituenten jeweils unabhängig Alkyl mit 1 bis 4 Kohlenstoffatomen, Halogen, Trihalomethyl, Alkoxy mit 1 bis 3 Kohlenstoffatomen, carboxylisches Acyl mit 1 bis 3 Kohlenstoffatomen, Carboxyl, (Alkoxy mit 1 bis 3 Kohlenstoffatomen)-carbonyl, Nitro, Cyano oder Di(alkyl mit 1 bis 3 Kohlenstoffatomen)-amino sind;

$R_1$ Wasserstoff, Halogen, Alkyl mit 1 bis 4 Kohlenstoffatomen, Trihalomethyl, Nitro, Cyano, Di-(alkyl mit 1 bis 4 Kohlenstoffatomen)-amino, (Alkoxy mit 1 bis 4 Kohlenstoffatomen)-carbonyl, Alkoxy mit 1 bis 4 Kohlenstoffatomen oder Hydroxyl ist;

$R_6$ und $R_7$ jeweils unabhängig Wasserstoff oder Methyl bedeuten;

m eine Zahl von 0 bis 3 ist; und

n eine Zahl von 2 bis 4 ist, mit der Maßgabe, daß n 3 oder 4 sein muß, wenn $R_1$ Wasserstoff oder 2-Methyl ist, $R_6$, $R_7$ und R Wasserstoff darstellen und m 0 ist;
oder ein nicht-toxisches, pharmazeutisch annehmbares Säureadditionssalz davon.

3. Eine Verbindung gemäß Anspruch 2, worin

R Wasserstoff oder

$$-\overset{\displaystyle O}{\overset{\|}{C}}-NH-A;$$

A Alkyl mit 1 bis 3 Kohlenstoffatomen; Cycloalkyl mit 5 bis 6 Kohlenstoffatomen; oder unsubstituiertes oder mono-, di- oder trisubstituiertes Phenyl ist, wobei die Substituenten jeweils unabhängig Alkyl mit 1 bis 4 Kohlenstoffatomen, Fluor, Chlor, Brom, Trifluormethyl, Methoxy, Acetyl oder Carbethoxy sind;

$R_1$ Wasserstoff, Fluor, Chlor, Brom, Alkyl mit 1 bis 4 Kohlenstoffatomen, Trifluormethyl oder Dimethylamino ist;

$R_6$ und $R_7$ jeweils unabhängig Wasserstoff oder Methyl sind;

m eine Zahl von 0 bis 3 ist; und

n eine Zahl von 2 bis 4 ist, mit der Maßgabe, daß n 3 oder 4 sein muß, wenn $R_1$ Wasserstoff oder 2-Methyl ist, $R_6$, $R_7$ und R Wasserstoff bedeuten und m 0 ist;
oder ein nicht-toxisches, pharmazeutisch annehmbares Säureadditionssalz davon.

4. Die Verbindung gemäß Anspruch 1, welche N-Cyclohexyl-S-{3-[4-(4-chlorbenzyl)-piperazin-1-yl]-propyl}-thiocarbamat-dihydrochlorid ist.

5. Die Verbindung gemäß Anspruch 1, welche 3-[4-(4-Chlorphenethyl)-piperazin-1-yl]-propanthiol-dihydrochlorid ist.

6. Die Verbindung gemäß Anspruch 1, welche N-(4-Methoxyphenyl)-S-{3-[4-(4-chlorphenethyl)-piperazin-1-yl]-propyl}-thiocarbamat-dihydrochlorid ist.

7. Die Verbindung gemäß Anspruch 1, welche N-(4-n-Butylphenyl)-S-{3-[4-[3-(4-chlorphenyl)-propyl]-piperazin-1-yl]-propyl}-thiocarbamat-dihydrochlorid ist.

8. Die Verbindung gemäß Anspruch 1, welche 3-[4-(4-Chlorbenzyl)-piperazin-1-yl]-propanthiol-dihydrochlorid ist.

9. Die Verbindung gemäß Anspruch 1, welche N-Phenyl-S-{3-[4-(4-chlorbenzyl)-piperazin-1-yl]-propyl}-thiocarbamat-dihydrochlorid ist.

10. Die Verbindung gemäß Anspruch 1, welche 2-[4-(4-Chlorbenzyl)-piperazin-1-yl]-ethanethiol dihydrochlorid ist.

11. Die Verbindung gemäß Anspruch 1, welche N-(4-Fluorphenyl)-S-{3-[4-(4-chlorbenzyl)-piperazin-1-yl]-propyl}-thiocarbamat-dihydrochlorid ist.

12. Die Verbindung gemäß Anspruch 1, welche N-(3,4,5-Trimethoxyphenyl)-S-{3-[4-(4-chlorphenethyl)-piperazin-1-yl]-propyl}-thiocarbamat-dihydrochlorid ist.

13. Die Verbindung gemäß Anspruch 1, welche N-Phenyl-S-{2-[4-(4-chlorbenzyl)-piperazin-1-yl]-ethyl}-thiocarbamat-dihydrochlorid ist.

14. Die Verbindung gemäß Anspruch 1, welche N-Cyclohexyl-S-{3-[4-(4-chlorphenethyl)-piperazin-1-yl]-propyl}-thiocarbamat-dihydrochlorid ist.

15. Die Verbindung gemäß Anspruch 1, welche N-(4-Methoxyphenyl)-S-{3-[4-[3-(4-chlorphenyl)-propyl]-piperazin-1-yl]-propyl}-thiocarbamat-dihydrochlorid-hemihydrat ist.

16. Die Verbindung gemäß Anspruch 1, welche N-(n-Hexyl)-S-{3-[4-(4-chlorbenzyl)-homopiperazin-1-yl]-propyl}-thiocarbamat-dihydrochlorid ist.

17. Ein immunologische, anti-allergische oder anti-inflammatorische Zusammensetzung, bestehend im wesentlichen aus einem inerten, pharmazeutischen Träger und einer wirksamen, immunologischen, anti-allergischen oder anti-inflammatorischen Menge einer Verbindung des Anspruch 1.

18. Die Verwendung einer Verbindung gemäß Anspruch 1 bei der Erzeugung einer pharmazeutischen Zusammensetzung, geeignet zur peroralen, parenteralen oder topischen Verabreichung zur Behandlung von immunologischen, allergischen oder entzündlichen Reaktionen in Warm-blütern.

19. Verfahren zur Herstellung einer Verbindung der Formel

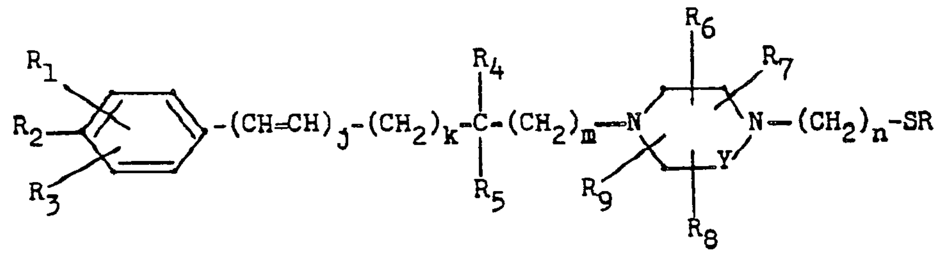

worin

R Wasserstoff oder

$$-\overset{\overset{\displaystyle O}{\|}}{C}-NH-A \text{ ist;}$$

A Alkyl mit 1 bis 8 Kohlenstoffatomen, Cycloalkyl mit 3 bis 7 Kohlenstoffatomen; oder unsubstituiertes oder mono-, di- oder trisubstituiertes Phenyl ist, wobei die Substituenten jeweils unabhängig Alkyl mit 1 bis 4 Kohlenstoffatomen, Halogen, Trihalomethyl, Alkoxy mit 1 bis 3 Kohlenstoffatomen, carboxylisches Acyl mit 1 bis 3 Kohlenstoffatomen, Carboxyl, (Alkoxy mit 1 bis 3 Kohlenstoffatomen)-carbonyl, Nitro, Cyano oder Di(alkyl mit 1 bis 3 Kohlenstoffatomen)-amino sind;

$R_1$, $R_2$ und $R_3$ jeweils unabhängig Wasserstoff, Halogen, Alkyl mit 1 bis 4 Kohlenstoffatomen, Trihalomethyl, Nitro, Cyano, Di-(alkyl mit 1 bis 4 Kohlenstoffatomen)-amino, (Alkoxy mit 1 bis 4 Kohlenstoffatomen)-carbonyl, Alkoxy mit 1 bis 4 Kohlenstoffatomen oder Hydroxyl sind;

$R_4$ und $R_5$ jeweils unabhängig Wasserstoff, Alkyl mit 1 bis 4 Kohlenstoffatomen oder Phenyl sind;

$R_6$, $R_7$, $R_8$ und $R_9$ jeweils unabhängig Wasserstoff oder Methyl sind;

Y $-CH_2-$ oder $-CH_2-CH_2-$ ist;

j 0 oder 1 ist;

k und m jeweils Zahlen von 0 bis 3 sind, wobei ihre Summe nicht mehr als 6 ist und 0 sein muß, wenn j 1 ist; und

n eine Zahl von 2 bis 4 ist, mit der Maßgabe, daß n 3 oder 4 sein muß, wenn $R_1$ Wasserstoff oder 2-Methyl bedeutet, $R_2$, $R_3$, $R_4$, $R_5$, $R_6$, $R_7$, $R_8$, $R_9$ und R Wasserstoff darstellen und j, k und m 0 sind und Y für $-CH_2-$ steht;

dadurch gekennzeichnet, daß eine Verbindung der Formel

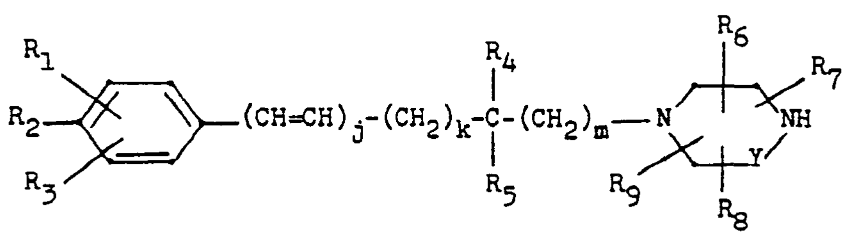

worin $R_1$, $R_2$, $R_3$, $R_4$, $R_5$, $R_6$, $R_7$, $R_8$, $R_9$, j, k, m und Y die vorstehend angegebenen Bedeutungen haben, mit einem Alkylhalogenid der Formel

$$X-(CH_2)_n-Z$$

worin X und Z identische oder verschiedene Halogene sind und n die vorstehend angegebene Bedeutung hat, umgesetzt wird, das so erhaltene Alkylhalogenid der Formel

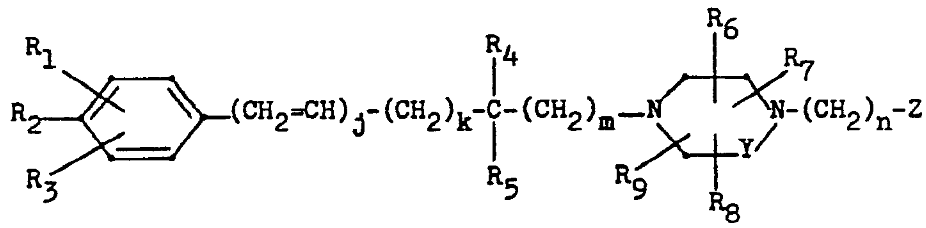

worin $R_1$, $R_2$, $R_3$, $R_4$, $R_5$, $R_6$, $R_7$, $R_8$, $R_9$, Y, Z, j, k, m und n die vorstehend angegebenen Bedeutungen haben, mit Thioharnstoff umgesetzt wird, das entstandene Isothiouroniumhalogenid-Zwischenprodukt der Hydrolyse unterworfen wird, um ein Endprodukt der Formel I zu erhalten, worin R Wasserstoff ist, und, falls gewünscht, Umsetzung dieses Endprodukts mit einem Isocyanat der Formel

$$A—N=C=O$$

worin A die vorstehend angegebene Bedeutung hat, zur Erzielung eines Endprodukts der Formel I, worin R —CO—NH—A ist.

**Revendications**

1. Composé de formule

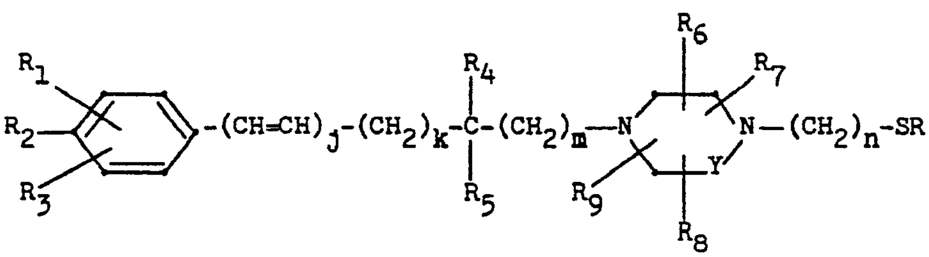

dans laquelle

R est hydrogène ou

$$-\overset{\overset{\displaystyle O}{\|}}{C}-NH-A;$$

A est alcoyle avec 1 à 8 atomes de carbone; cycloalcoyle avec 3 à 7 atomes de carbone; ou phényle non substitué ou mono-, di- ou tri-substitué, où les substituants sont chacun indépendamment alcoyle avec 1 à 4 atomes de carbone, halogène, trihalométhyle, alcoxy avec 1 à 3 atomes de carbone, acyle carboxylique avec 1 à 3 atomes de carbone, carboxyle, (alcoxy avec 1 à 3 atomes de carbone)carbonyle, nitro, cyano ou di(alcoyle avec 1 à 3 atomes de carbone)amino;

$R_1$, $R_2$ et $R_3$ sont chacun indépendamment hydrogène, halogène, alcoyle avec 1 à 4 atomes de carbone, trihalométhyle, nitro, cyano, di(alcoyle avec 1 à 4 atomes de carbone)amino, (alcoxy avec 1 à 4 atomes de carbone)carbonyle, alcoxy avec 1 à 4 atomes de carbone ou hydroxyle;

$R_4$ et $R_5$ sont chacun indépendamment hydrogène, alcoyle avec 1 à 4 atomes de carbone ou phényle;

$R_6$, $R_7$, $R_8$ et $R_9$ sont chacun indépendamment hydrogène ou méthyle;

Y est $—CH_2—$ ou $—CH_2—CH_2—$;

j est 0 ou 1;

k et m sont chacun des nombres entiers de 0 à 3, leur somme n'étant pas supérieure à 6 et devant être 0 lorsque j est 1; et

n est un nombre entier de 2 à 4, étant entendu que n doit être 3 ou 4 lorsque $R_1$ est hydrogène ou 2-méthyle, $R_2$, $R_3$, $R_4$, $R_5$, $R_6$, $R_7$, $R_8$, $R_9$ et R sont hydrogène et j, k et m sont 0 et Y est $—CH_2—$;

ou un sel d'addition d'acide pharmaceutiquement acceptable, non toxique de celui-ci.

2. Composé selon la revendication 1, qui a la formule

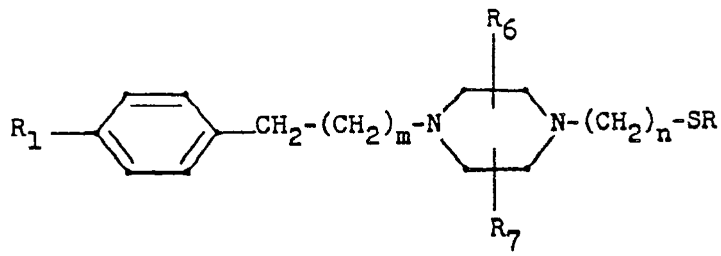

dans laquelle

R est hydrogène ou

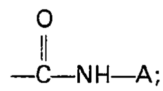

A est alcoyle avec 1 à 8 atomes de carbone; cycloalcoyle avec 3 à 7 atomes de carbone; ou phényle non substitué ou mono-, di- ou tri-substitué, où les substituants sont chacun indépendamment alcoyle avec 1 à 4 atomes de carbone, halogène, trihalométhyle, alcoxy avec 1 à 3 atomes de carbone, acyle carboxylique avec 1 à 3 atomes de carbone, carboxyle, (alcoxy avec 1 à 3 atomes de carbone)carbonyle, nitro, cyano ou di(alcoyle avec 1 à 3 atomes de carbone)amino;

$R_1$ est hydrogène, halogène, alcoyle avec 1 à 4 atomes de carbone, trihalométhyle, nitro, cyano, di(alcoyle avec 1 à 4 atomes de carbone)amino, (alcoxy avec 1 à 4 atomes de carbone)carbonyle, alcoxy avec 1 à 4 atomes de carbone ou hydroxyle;

$R_6$ et $R_7$ sont chacun indépendamment hydrogène ou méthyle;

m est un nombre entier de 0 à 3; et

n est un nombre entier de 2 à 4, étant entendu que n doit être 3 ou 4 lorsque $R_1$ est hydrogène ou 2-méthyle, $R_6$, $R_7$ et R sont hydrogène et m sont 0;

ou un sel d'addition d'acide pharmaceutiquement acceptable, non toxique de celui-ci.

3. Composé selon la revendication 2, ou R est hydrogène ou

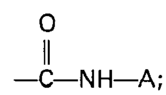

A est alcoyle avec 1 à 3 atomes de carbone; cycloalcoyle avec 5 à 6 atomes de carbone; ou phényle non substitué ou mono-, di- ou tri-substitué, où les substituants sont chacun indépendamment alcoyle avec 1 à 4 atomes de carbone, fluor, chlore, brome, trifluorométhyle, méthoxy, acétyle ou carbéthoxy;

$R_1$, est hydrogène, fluor, chlore, brome, alcoyle avec 1 à 4 atomes de carbone, trifluorométhyle ou diméthylamino;

$R_6$ et $R_7$ sont chacun indépendamment hydrogène ou méthyle;

m est un nombre entier de 0 à 3; et

n est un nombre entier de 2 à 4, étant entendu que n doit être 3 ou 4 lorsque $R_1$ est hydrogène ou 2-méthyle, $R_6$, $R_7$ et R sont hydrogène et m sont 0;

ou un sel d'addition d'acide pharmaceutiquement acceptable, non toxique de celui-ci.

4. Le composé selon la revendication 1, qui est le dichlorhydrate de N-cyclohexyl-S-{3-[4-(4-chlorobenzyl)pipérazine-1-yl]propyl}thiocarbamate.

5. Le composé selon la revendication 1, qui est le dichlorhydrate de 3-[4-(4-chlorophénéthyl)pipérazine-1-yl]propanethiol.

6. Le composé selon la revendication 1, qui est le dichlorhydrate de N-(4-méthoxyphényl)-S-{3-[4-(4-chlorophénéthyl)pipérazin-1-yl}thiocarbamate.

7. Le composé selon la revendication 1, qui est le dichlorhydrate de N-(4-n-butylphényl)-S-{3-[4-[3-(4-chlorophényl)propyl]pipérazine-1-yl]propyl}thiocarbamate.

8. Le composé selon la revendication 1, qui est le dichlorhydrate de 3-[4-(4-chlorobenzyl)pipérazine-1-yl]propanethiol.

9. Le composé selon la revendication 1, qui est le dichlorhydrate de N-phényl-S-{3-[4-(4-chlorobenzyl)pipérazine-1-yl]propyl}thiocarbamate.

10. Le composé selon la revendication 1, qui est le dichlorhydrate de 2-[4-(4-chlorobenzyl)-pipérazine-1-yl]éthanethiol.

11. Le composé selon la revendication 1, qui est le dichlorhydrate de N-(4-fluorophényl)-S-{3-[4-(4-chlorobenzyl)pipérazine-1-yl]propyl}thiocarbamate.

12. Le composé selon la revendication 1, qui est le dichlorhydrate de N-(3,4,5-triméthoxyphényl)-S-{3-[4-(4-chlorophénéthyl)pipérazine-1-yl]propyl}thiocarbamate.

13. Le composé selon la revendication 1, qui est le dichlorhydrate de N-phényl-S-{2-[4-(4-chlorobenzyl)pipérazine-1-yl]éthyl}thiocarbamate.

14. Le composé selon la revendication 1, qui est le dichlorhydrate de N-cyclohexyl-S-{3-[4-(4-chlorophénéthyl)pipérazine-1-yl]propyl}thiocarbamate.

15. Le composé selon la revendication 1, qui est le dichlorhydrate de N-(4-méthoxyphényl)-S-{3-[4-[3-(4-chlorophényl)propyl]pipérazine-1-yl]propyl}thiocarbamate, hémihydrate.

16. Le composé selon la revendication 1, qui est le dichlorhydrate de N-(n-hexyl)-S-{3-[4-(4-chlorobenzyl)homopipérazine-1-yl]propyl}thiocarbamate.

17. Composition immunologique, anti-allergique ou anti-inflammatoire consistant essentiellement en un support pharmaceutique inerte et en une quantité immunologique, anti-allergique ou anti-inflammatoire d'un composé selon la revendication 1.

18. Utilisation d'un composé tel que défini dans la revendication 1, dans la fabrication d'une composition pharmaceutique convenant pour l'administration pérorale, parentérale ou topique pour le traitement des réactions immunologiques, allergiques ou inflammatoires chez un animal à sang chaud.

19. Procédé pour la préparation d'un composé de formule

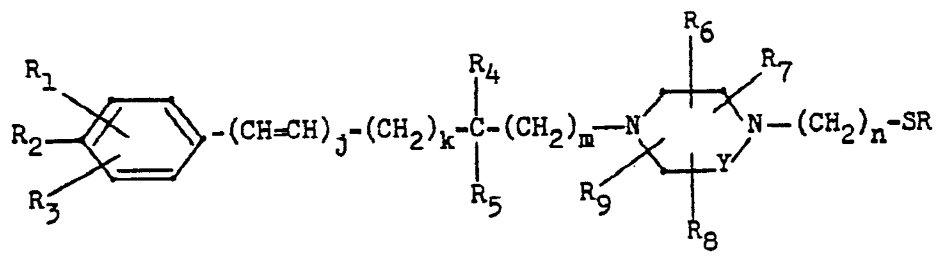

dans laquelle

R est hydrogène ou

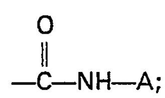

A est alcoyle avec 1 à 8 atomes de carbone; cycloalcoyle avec 3 à 7 atomes de carbone; ou phényle non substitué ou mono-, di- ou tri-substitué, où les substituants sont chacun indépendamment alcoyle avec 1 à 4 atomes de carbone, halogène, trihalométhyle, alcoxy avec 1 à 3 atomes de carbone, acyle carboxylique avec 1 à 3 atomes de carbone, carboxyle, (alcoxy avec 1 à 3 atomes de carbone)carbonyle, nitro, cyano ou di(alcoyle avec 1 à 3 atomes de carbone)amino;

$R_1$, $R_2$ et $R_3$ sont chacun indépendamment hydrogène, halogène, alcoyle avec 1 à 4 atomes de carbone, trihalométhyle, nitro, cyano, di(alcoyle avec 1 à 4 atomes de carbone)amino, (alcoxy avec 1 à 4 atomes de carbone)carbonyle, alcoxy avec 1 à 4 atomes de carbone ou hydroxyle;

$R_4$ et $R_5$ sont chacun indépendamment hydrogène, alcoyle avec 1 à 4 atomes de carbone ou phényle;

$R_6$, $R_7$, $R_8$ et $R_9$ sont chacun indépendamment hydrogène ou méthyle;

Y est $-CH_2-$ ou $-CH_2-CH_2-$;

j est 0 ou 1;

k et m sont chacun des nombres entiers de 0 à 3, leur somme n'étant pas supérieure à 6 et devant être 0 lorsque j est 1; et

n est un nombre entier de 2 à 4, étant entendu que n doit être 3 ou 4 lorsque $R_1$ est hydrogène ou 2-méthyle, $R_2$, $R_3$, $R_4$, $R_5$, $R_6$, $R_7$, $R_8$, $R_9$ et R sont hydrogène et j, k et m sont 0 et Y est $-CH_2-$;

qui comprend les étapes consistant à faire réagir un composé de formule

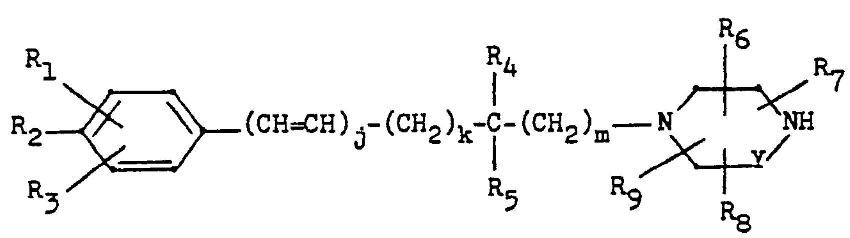

dans laquelle $R_1$, $R_2$, $R_3$, $R_4$, $R_5$, $R_6$, $R_7$, $R_8$, $R_9$, j, k, m et Y ont les significations définies précédemment, avec un halogénure d'alcoyle de formule

$$X—(CH_2)_n—Z$$

dans laquelle X et Z sont des halogènes identiques ou différents, et n a les significations définies précédemment, à faire réagir l'halogénure d'alcoyle de formule

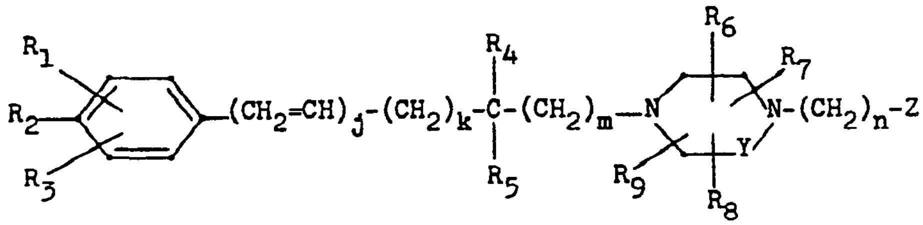

dans laquelle $R_1$, $R_2$, $R_3$, $R_4$, $R_5$, $R_6$, $R_7$, $R_8$, $R_9$, Y, Z, j, k, m et n ont les significations définies précédemment ainsi obtenues avec la thiourée, à soumettre l'halogénure d'isothiouronium intermédiaire résultant à une hydrolyse pour obtenir un produit final de formule I dans laquelle R est hydrogène, et si on le désire, à faire réagir ledit produit final avec un isocyanate de formule

$$A—N=C=O$$

dans laquelle A a les significations définies précédemment, pour obtenir un produit final de formule I dans laquelle R est —CO—NH—A.